# EUROPEAN PATENT SPECIFICATION

(11) **EP 1 996 230 B1**
(45) Date of publication and mention of the grant of the patent: **30.04.2014**
(21) Application number: 07722975.5
(22) Date of filing: 28.02.2007
(51) Int. Cl.: A61K 39/395, C07K 19/00, A61P 37/08, C12N 15/63, C07K 16/28, C07K 16/46, C07K 14/00

(54) **BISPECIFIC MOLECULE BINDING TLR9 AND CD32 AND COMPRISING A T CELL EPITOPE**
TLR9 UND CD32 BINDENDES UND EIN T-ZELLEN-EPITOP UMFASSENDES BISPEZIFISCHES MOLEKÜL
MOLÉCULE BISPÉCIFIQUE SE LIANT À TLR9 ET À CD32 ET COMPRENANT UN ÉPITOPE DE LYMPHOCYTE T

(30) Priority: 03.03.2006 EP 06110672
(43) Date of publication of application: 03.12.2008
(73) Proprietor: S-TARget therapeutics GmbH, 1190 Vienna (AT)
(72) Inventor: MUDDE, Geert, 2384 Breitenfurt bei Wien (AT); HIMMLER, Gottfried, 2301 Gross-Enzersdorf (AT)
(74) Representative: Redl, Gerda
(86) International application number: PCT/EP2007/001722
(87) International publication number: WO 2007/098934

(56) References cited:
- WO-A-97/07218
- WO-A-03/035695
- ROTHENFUSSER S ET AL: "Plasmacytoid dendritic cells: The key to CpG" HUMAN IMMUNOLOGY 01 DEC 2002 UNITED STATES, vol. 63, no. 12, 1 December 2002 (2002-12-01), pages 1111-1119, XP009071925 ISSN: 0198-8859 cited in the application
- HOREJS-HOECK J ET AL: "Inhibition of immunoglobulin E synthesis through Fc[gamma]RII (CD32) by a mechanism independent of B-cell receptor co-cross-linking" IMMUNOLOGY 2005 UNITED KINGDOM, vol. 115, no. 3, 2005, pages 407-415, XP009071924 ISSN: 0019-2805
- WANG ET AL: "Antigen targeting to dendritic cells with bispecific antibodies" JOURNAL OF IMMUNOLOGICAL METHODS, ELSEVIER SCIENCE PUBLISHERS B.V.,AMSTERDAM, NL, vol. 306, no. 1-2, 30 November 2005 (2005-11-30), pages 80-92, XP005197266 ISSN: 0022-1759
- BHEEKHA ESCURA R ET AL: "REGULATION AND TARGETING OF T-CELL IMMUNE RESPONSES BY IGE AND IGG ANTIBODIES" IMMUNOLOGY, BLACKWELL PUBLISHING, OXFORD, GB, vol. 86, no. 3, 1995, pages 343-350, XP009030554 ISSN: 0019-2805
- BJERSING J L ET AL: "Synergistic action of immunostimulatory DNA and Fcgamma receptor IIB-crosslinking on B-cell phenotype and function" IMMUNOBIOLOGY, FISCHER, STUTTGART, DE, vol. 210, no. 1, 6 July 2005 (2005-07-06), pages 23-32, XP004944310 ISSN: 0171-2985
- MEANS TERRY K ET AL: "Human lupus autoantibody-DNA complexes activate DCs through cooperation of CD32 and TLR9.", THE JOURNAL OF CLINICAL INVESTIGATION FEB 2005 LNKD- PUBMED:15668740, vol. 115, no. 2, February 2005 (2005-02), pages 407-417, ISSN: 0021-9738

## Description

The present invention relates to molecules with binding specificity to both, Toll-like Receptor 9 (TLR9) and CD32 containing one or more T cell antigen epitopes.

The invention further relates to the production of these molecules and their use for the preparation of medicaments for the treatment of allergies.

### Introduction:

Allergy is considered to be a hypersensitive reaction to proteins in the environment (air/water/food), Allergens are antigens to which atopic patients respond with IgE antibody responses subsequently leading to allergic reactions. Antigens in the complexes or fusion proteins can be environmental allergens (e.g. house dust mite, birch pollen, grass pollen, cat antigens, cockroach antigens), or food allergens (e.g. cow milk, peanut, shrimp, soya), or a combination of both. IgE molecules are important because of their role in effector cell (mast cell, basophiles and eosinophiles) activation. More recently, it has been accepted that IgE also plays an important role in the induction phase of allergic diseases, by up-regulating the antigen capture potential of B cells and dendritic cells (DC), both through low affinity (CD23) and high affinity receptors (FcεRI) [1]. The negative functions of IgE antibodies can be counteracted by allergen specific IgG antibodies, e.g. because they direct the immune response away from B cells to monocytes and DC [2]. In addition, they compete with IgE molecules for allergen binding sites. Allergies therefore can be treated, cured and prevented by the induction of allergen specific IgG molecules.

IgG molecules have a serum half-life of approximately 3 weeks as compared to roughly 3 days for IgE molecules. IgE molecules are induced by the interaction between (naïve) B cells and Th2 cells which provide the IL-4 and IL-13 together with CD40L expression necessary to induce a class switch to IgE in memory B cells and plasma cells [3]. In contrast, Th1 cells, which produce IFN-γ and IL-2, induce a class switch to IgG. Therefore, induction of Th1, rather than Th2 helper T cell responses against allergens, is beneficial for the prevention, treatment and cure of allergic diseases.

To date several forms of active vaccination using allergens are used. The most common is the so called "Immunotherapy", which depends on frequent immunizations with relatively high concentrations of allergens. This technique is only moderately effective in a minority of allergic diseases such as Bee venom allergy and in some cases of Rhinitis and Conjunctivitis, and recently some reports have shown effectiveness in asthma and atopic dermatitis. More recently rush immunotherapy, where increasing amounts of allergen are injected in a rather short time frame, has been proposed with slightly better results [4; 5]. Usually the subcutaneous route is used for administration of the allergens, but recently this route has been compared to oral application or even local application, the results are generally positive but not allays consistent. A different technique for immunotherapy is the one described by Saint-Remy (EP 0 178 085 and 0 287 361), which makes use of autologous IgG antibodies which are in vitro complexed to the relevant allergens. This technique allows far smaller amounts of allergen to be applied with fewer side effects.

The mechanism behind these therapies is unclean. In the classical therapy there seems to be a beneficial effect if the therapy induces an increase in specific IgG antibodies, although not every significant increase of specific IgG is correlated with successful immunotherapy A possible argument why this is the case is the relatively low affinity of IgG antibodies for CD32 on B cells, monocytes and mast cells. The Saint-Remy approach selects the specific IgG antibodies from the patient, which are subsequently mixed with relevant allergens in vitro. This way they assure that the allergen cannot react freely with cells or other antibody isotopes on cells such as IgE on mast cells. In addition they claims that anti-idiotypic antibodies are raised against the specific IgG molecules, which in the future will prevent allergy.

In WO 97/07218 Allergen-anti-CD32 Fusion Proteins are described. In this publication the problems with isolating specific IgG molecules and the low affinity of these IgG antibodies for CD32 are circumvented and the risk factors of classical immunotherapy, which uses complete "IgE binding" allergens, are reduced. However, the claimed induction of Th1 memory responses due to solely directing the anti-CD32 containing vaccine to dendritic cells cannot be substantiated.

Means Terry et al. (The Journal of Clinical Investigation 2005, vol 115(2): 407-417) describe that DNA-containing immune complexes within lupus serum stimulate plasmacytoid DCs to produce cytokines via a cooperative interaction between Toll-like receptor 9 and FcgammaRIIa (CD32).

Even in view of the intensive research for therapeutic approaches to treat allergic diseases, there is still a great demand for providing medicaments for successful treatment of allergies.

The object of the invention is therefore to provide novel molecules with improved properties for the treatment of allergic diseases.

According to the invention this object is achieved by the subject matter of the claims.

### Brief description of the invention :

### Background:

CD32 is strongly expressed on monocytes/dendritic cells and B cells and thus the molecule of the present invention is designed to direct the immune response to these important immunological cells, with the intention to prevent allergen presentation by the B cells, while promoting allergen presentation by especially dendritic cells (DCs), the latter leads to induction of Th1 responses against the allergens in the molecule or molecule complex that can be formulated as vaccine. More recent knowledge shows that two types of dendritic cells (DC) exist myeloid (mDC) and plasmacytoid dendritic cells (pDC) [6], which has led to the new concept of DC1 and DC2 cells [7]. In this concept DC1 cells promote the induction of Th1 cell development after antigen specific stimulation and DC2 cells support the development of Th2 cells. Monocyte derived DC (or mDC) are generally considered to be of DC1 type, whereas pDC are considered to be DC2 type [6]. Both types of DC express CD32a and will induce an allergen specific T cell response; however it is not guaranteed that the outcome will be of The type. In fact, in allergic donors Th2 responses are more likely [8]. Importantly, the pDC express the TLR9 receptor, which binds CpG-ODNs (oligodeoxynucleotides [ODNs] containing unmethylated CpG motifs). Activation of this receptor in the pDC leads to a very strong production of IFN-α and IL-12 [9], which promotes Th1 induction and thus transforms the potential DC2 into DC1 cells.

Therefore, the molecule of the invention can combined the activation of the TLR9 receptor in pDC with the specific stimulation and induction of allergen specific Th1 cells and comprises therefore a significant improvement of earlier concepts.

The invention comprises a pharmaceutical preparation comprising a molecule or a molecule complex having binding specificity for toll-like receptor 9 and CD32, wherein the molecule or molecule complex includes at least one epitope, preferably at least one T cell epitope, of at least one antigen , and wherein the CD32 binding part is selected from the group consisting of antibodies, antibody fragments, protein domains and peptides, and the TLR9 binding part is a nucleic acid.

The molecule or molecule complex of the invention will also bypass the effector function of mast cells, which carry IgE, for the native allergen of which T cell epitopes have been selected to be part of the fusion protein.

Preferably the molecule or molecule complex according to the invention can have one or more of the following unique characteristics:
- Activation and induction of allergen specific Th1 cells, without activation of allergen- specific B-cells.
- Activation and induction of allergen specific Th1 cells, without activation of mast cells or any other effector cell, which, by means of allergen specific IgE or IgG, may become activated by the natural allergens of which the selected T cell epitopes are represented in the molecule or molecule complex of the invention.

The CD32-binding part of the molecule or molecule complex of the invention selects the relevant cells, which should internalize the complete molecule or molecule complex.

After internalization of the fusion protein according to the present invention by antigen presenting cells the molecule of the invention is degraded and various peptides, in cluding the incorporated T cell epitope(s) are presented on the MHC class II molecules of the antigen presenting cells, therefore stimulating allergen specific T cells.

The incorporated TLR9-binding structure(s) in the molecule or molecule complex of the invention are necessary for the induction of an allergen specific Th1 memory pool, by binding to the cytoplasmatic [10;11] TLR9 receptor. Activation of the TLR9 receptor leads to a strong induction of IFN-α and IL12 production [9].

According to the present invention, a molecule is a single entity made up of atoms and/or other molecules by covalent bonds. The molecule can be made up of one single class of substances or a combination thereof. Classes of substances are e.g. polypeptides, carbohydrates, lipids, nucleic acids etc.

A molecule complex is an aggregate of molecules specifically and strongly interacting with each other. A complex of various molecules may be formed by hydrophobic interactions (such as e.g. the binding of antibody variable regions in an Fv) or by strong binding of one molecule to another via ligand/receptor interactions such as antibody-antigen binding or avidin-biotin or by complex formation via chelating chemical groups and the like.

An Antigen can be a structure which can be recognized by an antibody, a B-cell-receptor or a T-cell-receptor when presented by MHC class I or II molecules.

An epitope is the smallest structure to be specifically bound within by an antibody, a B-cell-receptor or a T-cell receptor, when presented by MHC class I or II molecules Specificity is defined as preferred binding to a certain molecular structure (in antibody/antigen interactions also called epitope) within a certain context.

A domain is a discrete region found in a protein or polypeptide. A monomer domain forms a native three-dimensional structure in solution in the absence of flanking native amino acid sequences. Domains of the invention will specifically bind to CD32 and/or TLR9 and/or display or present epitopes. Domains may be used as single domains or monomer domains or combined to form dimers and multimeric domains. For example, a polypeptide that forms a three-dimensional structure that binds to a target molecule is a monomer domain.

According to the present invention the term antibody includes antibodies or antibody derivatives or fragments thereof as well as related molecules of the immunoglobulin superfamily (such as soluble T-cell receptors). Among the antibody fragments are functional equivalents or homologues of antibodies including any polypeptide comprising an immunoglobulin binding domain or a small mutated immunoglobulin domain or peptides mimicking this binding domain together with an Fc region or a region homologous to an Fc region or at least part of it. Chimeric molecules comprising an immunoglobulin binding domain, or equivalents, fused to another polypeptide are included.

Allergens are antigens to which atopic patients respond with allergic reactions.

### Detailed description of the invention

The invention provides a pharmaceutical preparation comprising a molecule or molecule complex capable of binding to TLR9 and to CD32, comprising at least one epitope of at least one antigen, wherein the CD32 binding part of the molecule or molecule complex is selected from the group consisting of antibodies, antibody fragments or peptides, and the TLR9 binding part is a nucleic acid.

In one embodiment of the invention the molecule or molecule complex comprises at least three parts, one part being a structure specifically binding to TLR9 (monovalently, bivalently or multivatently), another part being a structure specifically binding to CD32 (monovalently, bivalently or multivatently) and at least one other part being one or more T cells epitopes of an antigen and/or allergen. The parts may be independent structures which are linked together either by chemical linkages or by genetic fusion or by other (non-covalent) interactions such as ligand-receptor or antibody interactions.

The linkages between the different parts may be different. For example, in one preferred embodiment, the linkage between the parts binding to TLR9 and CD32 is by genetic fusion and the link to at least one of the T cell epitopes is via a receptor/ligand interaction (e.g. biotin-streptavidin). The advantage of such a setup is the flexibility in production. The bispecific (anti-TLR9/anti-CD32), generic part of the molecule complex can be produced in the same way for all patients, selected T cell epitopes are linked to the generic part of the molecule complex according to the need. The selection can be based on disease prevalence or on results of individual specificity tests of patients (specific allergy). The complex formation may be performed centralized or at the bed side or at a physicians office.

Chemical linkage of molecules of the various binding molecules of the same or different chemical class may be achieved by many different techniques yielding either a defined molecular ratio of the various parts of the molecule or molecule complex of the invention. It may also lead to a mixture of molecules with different molecular ratios of the various parts of the molecule or molecule complex of the invention.

The ratio of the various parts of the invention may be an equimolar or non-equimolar. The molecule may be monovalent for binding to TLR9 and/or CD32 and/or T cell epitope(s). It may also be bi-, tri- and multivalent for at least one of the parts of the molecule or the molecule complex. If the binding to TLR9 and/or CD32 is bivalent or of higher valency, the binding specificity may be for one or for more epitopes on CD32 and/or TLR9 respectively.

In another embodiment of the invention the binding specificities of the molecule are overlapping so that one part of the molecule or the molecule complex of the invention is binding to both, TLR9 and CD32. Such a part could be selected for by simultaneous screening of molecules for binding to both CD32 and TLR9 or by engineering of a molecule to bind both, CD32 and TLR9. For example, a protein scaffold can be used for displaying loops to bind CD32 and other loops that bind to TLR9.

In a further embodiment of the invention, a protein scaffold can be used to display structures that bind CD32, structures that bind TLR9 and to display T-cell epitopes.

The specific binding molecules can be natural ligands for CD32 and TLR9 and derivatives thereof. For example, the Fc-part of immunoglobulin is binding to CD32. CpG is a naturally occurring ligand for TLR9.

The specific binding molecules can be peptides. CD32- and TLR9-specific peptides according to the invention can be selected by various methods such as phage display technology or by screening of combinatorial peptide libraries or peptide arrays. The peptides can be selected and used in various formats such as linear, constrained or cyclic peptides, the peptides can be chemically modified for stability and/or specificity.

A specifically binding peptide may also be derived from analysis of interaction of a naturally occurring proteinaceous ligand to TLR9 and CD32 by isolation of the minimal binding site of the ligand.

The specific binding peptides can be used as such in the molecule or the molecule complex of the invention or used to be incorporated into other structures such as by grafting into protein scaffolds, antibodies and protein domains or chemically coupled to carrier molecules which might be part of the molecule or molecule complex of the invention.

The CD32-binding part of the molecules or molecule complex of the invention can be comprised of proteins such as antibodies or antibody fragments (such as Fab, Fv, scFv, dAb, F(ab)₂, minibody, small mutated immunoglobulin domains, soluble T-cell receptor, etc). Antibodies and antibody fragments and derivatives may be generated and selected for binding to CD32 according to known methods such as hybridoma technology, B-cell cloning, phage display, ribosome display or cell surface display of antibody libraries, array screening of variant antibodies.

The binding parts of the molecules or molecule complexes of the invention can be protein domains which occur naturally or domains which are artificially modified. Protein domains or domain derivatives, e.g domains with mutations such as amino acid substitutions, deletions or insertions or chemically modified domains may be selected for binding to CD32 according to known methods (e.g. phage and cell surface display of libraries of domains or domain variants and screening, arrays of variant molecules and screening).

In a preferred embodiment, the binding part of a molecule or molecule complex of the invention comprises a small mutated immunoglobulin domain (SMID) as described in PCT/EP2006/050059.

The binding part of the molecule or molecule complex of the invention can be nucleic acids such as RNAs or DNAs which can be selected for specific binding to TLR9 according to known methods such as aptamer screening and in vitro evolution techniques.

A preferred embodiment of the invention is a recombinant fusion protein consisting of at least one epitope of at least one antigen, at least one binding site interacting with TLR9 and at least one binding site interacting with CD32. The antigen can be as small as one T cell epitope from one antigen or can be a cocktail or mixture of one or more T cell epitopes from one or more different antigens fused or linked together in a way that allows proper processing and presentation by MHC molecules. The order of the epitopes can be selected according to different criteria such as product stability effective processing, (non-)recognition by preformed antibodies in the treated persons. Generally one will select for a stable molecule which can be efffciently presented by MHC and which will lead to minimal recognition by preformed antibodies.

The invention further comprises the physical coupling of at least one molecule interacting with TLR9, at least one molecule interacting with CD32 and one or more T cell epitopes from one or more antigens linked together in a random form.

Additionally, the invention provides the preparation of a medicament containing the fusion protein according to the invention and its use for treatment of allergies. The medicament can be a vaccine formulation containing the molecule or molecule complex according to the invention, useful esp. for active immunotherapy.

The recombinant production of bispecific binding structures of the molecule or the molecule complex of the invention (i.e. binding to CD32 and to TLR9) can be accomplished in different ways, e.g. by
- Quadroma technology (fused hybridomas) [12;13]
- bispecific scFvs, either as "diabodies" or simply by genetic fusion of different scFvs [14]
- single-domain antibodies in which VH recognizes one antigen and VL another one
- chi-bAbs (as described in EP0640130)
- small mutated immunoglobulin domains, by including engineered immunoglobulin domains, specifically binding to CD32 and/or to TLR9 in constructs coding either for complete antibodies or for antibody fragments such as Fab (according to PCT/EP2006/050059)
- in the context of this invention, binding to CD32 can also be accomplished by monomeric or multimeric immunoglobulin Fc region(s) or a parts thereof especially when the affinity for CD32 of the Fc parts is enhanced, while TLR9 binding is achieved through the normal binding site (VH/VL) of the antibody
- Fc-region(s) of an immunoglobulin or parts thereof, binding to CD32, fused to any other TLR9 specific binding motif
- the Fc part of the above mentioned antibody may be "glyco-engineered " to increase the affinity for human FcyR's [15]
engineered scaffolds, specifically binding to TLR9 and/or CD32 of any kind can be used and linked together as needed. These binding scaffolds can be protein domains, fibronectin III, lipocalins, Protein A or α-amylase inhibitor, Ankyrin Repeat Proteins, a C2 domain, an A-domain, an EGFR like domain, a dab, a chi-bAb, CTLA-4, gamma crystalline or any other protein, protein domain or part thereof.

The molecule or molecule complex of the invention consist of one or more epitopes and one or more binding structures, which interact with TLR9, preferably human TLR9 and one or more binding structures, which interact with CD32, preferable human CD32. For easy in vivo testing of the inventive protein the binding structures that recognize human TLR9 and human CD32 may cross react with monkey or mouse TLR9 and monkey or mouse CD32. The selected antigens/allergens may be complete natural/native proteins or parts of these, as long as epitopes which can be presented on MHC class II molecules and which can be recognized by T cells are present on the sequences present in the molecule or molecule complex. The part(s) of the molecule or molecule complex, which interact with TLR9 and CD32 may be complete or incomplete (modified) antibodies or fragments or derivatives thereof, as long as binding to TLR9 and CD32 is retained.

Alternatively, anti-TLR9 and anti-CD32 antibodies or derivatives or fragments thereof, which still specifically recognize and bind to human TLR9 and CD32 such as expressed by B cells, and dendritic cells can be used.

Alternatively, the antibodies interacting with TLR9 or CD32 are improved antibodies with higher affinity than the original antibodies.

Exemplary antibody molecules are intact immunoglobulin molecules and those portions of an immunoglobulin molecule that contains the paratope, including those portions known as Fab, Fab', F(ab')₂,Fcand F(v), dAb.

The antibodies can be IgG, IgM, IgE, IgA or IgD. The molecules interacting with TLR9 or CD32 can be of any origin, preferably of mammalian origin, preferably of human, mouse, camel, dog or cat origin or humanized. Preferably the molecules are antibodies, preferably human or humanized antibodies.

As used herein, if the molecule or molecule complex of the invention is a fusion protein, it can be expressed in host cells which cover any kind of cellular system which can be modified to express the fusion protein. Within the scope of the invention, the term "cells" means individual cells, tissues, organs, insect cells, avian cells, mammalian cells, hybridoma cells, primary cells, continuous cell lines, stem cells and/or genetically engineered cells, such as recombinant cells expressing an antibody according to the invention.

Cells can be bacterial cells, fungal cells yeast cells, insect cells, fish cells and plant cells.

Preferably the cells are animal cells, more preferably mammalian cells. These can be for example BSC-1 cells, LLC-MK cells, CV-1 cells, CHO cells, COS cells, PerC6 cells, murine cells, human cells, HeLa cells, 293 cells, VERO cells, MBBK cells, MDCK cells, MDOK cells, CRFK cetts, RAF cells, TCMK cells, LLC-PK cells, PK15 cells, WI-38 cells, MHC-5 cells, T-FLY cells, BHK cells, SP2/0. NS0 cells or derivatives thereof.

Preferably the binding structures of the molecule or the molecule complex of the invention recognizing CD32 are small mutated immunoglobulin domains, being for example a complete antibody in Which one binding site is formed by VH/VL, and is combined with a second binding site which can be an engineered CL, CH1, CH2, CH3, CH4, VL or VH domain according to PCT/EP/2006/050059.

According to the invention, the molecule or molecule complex contains at least one structure that specifically binds to CD32.

An anti-CD32 antibody can be derived by known methods (such as hybridoma technology, B-cell cloning and antibody library screening). For selection, cells displaying CD32 in a natural format can be used or a recombinant extracellular part of CD32 can be used or synthetic peptides selected from the CD32 amino acid sequence can be used. Selection criteria are that the binding structure recognizes CD32a. In case also OD32b is recognized it is preferred that the affinity for CD32a ≥ CD32b

As an example, the Fab fragment from the anti-CD32 IV.3 antibody derived from the cell line HB-217 can be used. Using the method e.g. described by Orlandi et al¹⁶, the Fab fragment is cloned from the cell line HB-217. Alternatively, other formats such as scFv can be constructed of the known V-gene sequences. However, for optimal combination with an anti-TLR9 antibody or Fab fragment or Fv fragment it is preferred to select specific binders using one or more of the small mutated immunoglobulin domain libraries from CH1, CH2 CH3, CH4, CL, VL or VH. Selected CH1, CH2, CH3, CH4, CL, VL or VH domains can then be cloned into the existing sequence of an anti-TLR9 antibody or a Fab or an Fv fragment thereof thus generating a bi-specific antibody or Fab fragment.

The selected CD32 binding entities should preferably have the following characteristics:
1. Interaction with CD32a leads to internalization of the receptor-binding-structure complex, activation of the antigen presenting cell through the ITAM motiv in the cytoplasmic tail of the receptor and antigen presentation of the linked/fused T cell epitopes
2. Interaction with CD32b leads to negative signaling of the receptor through the ITIM motiv
3. Interaction should show cross reactivity between human and monkey CD32 (for testing of efficacy in a relevant in vivo model)
4. interaction should show cross reactivity with mouse CD32 (for testing in an established in vivo model for allergy)

For obtaining a binding structure that specifically binds to TLR9, several procedures can be used (such as hybridoma technology, B-cell cloning and antibody library screening). For selection, cells expressing TLR9 in a natural format can be used to isolate natural TLR9 or a recombinant TLR9 can be used or synthetic peptides selected from the TLR9 amino acid sequence can be used. Alternatively, purified TLR9 or TLR9 expressed by cell lines can be used. Antibody genes coding for VL and VH respectively can be extracted after selection for binding to TLR9 and be used to design a recombinant antibody or Fab fragment specific for human TLR9. Alternatively, a single-chain Fv can also be made and fused with the anti-CD32 scFv mentioned above. However, for optimal combination with the anti-CD32 antibody or scFv or Fab fragment it is preferred to select specific binders using one or more of the small mutated immunoglobulin domain libraries from CH1, CH2 CH3, CH4, CL, VH or VL. Selected CH1, CH2, CH3, CH4, CL, VL or VH domains can then be cloned into the existing antibody or Fab fragment or scFv of anti-CD32 antibody thus generating a bi-specific Fab fragment. The selected TLR9 binding entities should preferably have the following characteristics:
1. Interaction with TLR9 leads to signal transduction and cytokine production
2. Interaction may show cross reactivity between human and monkey TLR9 (for testing of efficacy in a relevant in vivo model)
3. Interaction may show cross reactivity with mouse TLR9 (for testing in an established in vivo model for allergy) and CD32

Of course the fusion protein can similarly be made using the Fab part of an existing aTRL9 monoclonal antibody. Using the method e.g. described by Orlandi et al¹⁶, the Fab fragment is cloned from e.g. clone 26C593 available from Imgenex Corp., as decribed above for the fab fragment of the aCD32 Ab IV.3. Again for optimal combination with the anti-TLR9 Fab fragment it is best to select specific binders for CD32 using one or more of the small mutated immunoglobulin domain libraries from CH1, CH2, CH3, CH4, CL, VL or VH. Selected CH1, CH2, CH3, CH4, CL, VL or VH domains can then be cloned into the existing Fab fragment of anti-TLR9 antibody thus generating a bi-specific molecule.

Finally, e.g. in the absence of available suitable existing Ab's for both CD32 and TLR9, it is also possible to construct a bi-specific molecule using the small mutated immunoglobulin domain libraries from CH1, CH2 CH3 or CL to select specific binders for both CD32 and TLR9 which are subsequently combined to form new structures existing of at least 1 binding structure specific for CD32 and 1 binding structure specific for TLR9 derived from any of the possible libraries in any of the possible combinations (CH1-CH1 or CH1-CH2 or CH1 CH3 or CH2-CH4, or CH3-CH4, or CH1-CH4 or or CH2-CH3 etc).

Alternatively, a single variable domain of the immunoglobulin superfamily may be selected for binding to TLR9 or CD32 with CDR-loops. The selected binder is then randomized at non-structural loop positions to generate a library of variable domains which is selected for the respective other antigen, ie. in case of a variable domain binding with CDR loops to TLR9 the selection is for binding to CD32 and vice versa. It is also possible to select a library of a V-domain which contains variations in the CDR loops at the same time as variations in the non-CDR-loops for binding to TLR9 and CD32 sequentially or simultaneously.

Such bispecific V-domains may also be part of antibodies or antibody fragments such as single-chain-Fvs, Fabs or complete antibodies.

Selection of a suitable TLR9 epitope:
Sequence 244-256 (SEQ ID No 1) of the mature TLR9 protein in amino acid 1 letter code: will fulfill criterion 1 and 2 but not 3, whereas
Sequence 176-191 (SEQ ID No 2) of the mature protein TLR9 in amino acid 1 letter code and
Sequence 216-240 of the mature protein TLR9 (SEQ ID No 3) in amino acid 1 letter code
will fulfill all three criteria and are thus preferred for use in this invention.

The process for producing the molecule or molecule complex is carried out according to known methods, e.g. by using recombinant cloning techniques or by chemical cross linking.

A product as described in this invention can be produced in the following way:

The obtained VH and VL of the anti-CD32 antibody are fused to CH1 and CL respectively. The CL has previously been engineered using SMID technology (PCT/EP2006/050059) and selected using phage display to bind to TLR9 as described below. CH1 is fused at its C-terminus to a sequence encoding the selected T cell epitopes. These two fusion-protein encoding genes are cloned into an expression vector allowing the expression of two independent genes (or into two independent expression vectors) and are co-expressed in bacteria, yeast or animal cells or any other suitable expression system. Thus, an Fab with the desired characteristics, i.e. binding to CD32, binding to TLR9 and carrying the relevant T-cell epitopes is produced.

Alternative examples applying SMID technology:
- An scFv against TLR9 is derived from a phage display library or from an existing hybridoma, and a CD32 binding molecule is derived from a CH2-CH4, or CH3-CH4, or CH1-CH4 or small mutated immunoglobulin domain library. These two coding sequences are ligated together and a sequence coding for T cell epitopes is attached. The fusion protein is then expressed in bacteria, yeast or animal cells or any other suitable expression system
- Alternatively, TLR9-specificity and CD32-specificity are swapped: An scFv against CD32 is derived e.g. from a phage display library or from an existing hybridoma, and a TLR9-binding molecule is derived from a CH2-CH4, or CH3-CH4, or CH1-CH4 or small mutated immunoglobulin domain library. These two coding sequences are ligated together and a sequence coding for T cell epitopes is attached. The fusion protein is then expressed in bacteria, yeast or animal cells or any other suitable expression system
- VH and VL of an anti-TLR9 antibody are fused to CH1 and CL respectively. CL has previously been engineered and selected using phage display to bind to CD32 (SMID). CH1 is fused at its C-terminus to a sequence encoding the T cell epitopes. These two fusion-protein encoding genes are cloned into an expression vector allowing the expression of two independant genes (or into two independent expression vectors) and are coexpressed in bacteria, yeast or animal cells or any other suitable expression system. (again, anti-TLR9 and anti-CD32 can be swapped. CH1 and CL can also be swapped)
- Heavy and light chain genes of an anti-TLR9 antibody are taken as a whole. In the heavy chain gene, the CH2 (or CH1 or CH3 or CH4) region is replaced by a CH2 (or CH1 or CH3 or CH4 or CL or VH or VL) region which has previously been engineered and selected using phage display to bind to CD32 (small mutated immunoglobulin domain). CH1, CH2, CH3 or CH4 is fused at its C-• terminus to a sequence encoding the T cell epitopes. These two genes are again cloned in expression vectors and expressed in animal cells.
- 2 small mutated immunoglobulin domains, one specific for TLR9, the other specific for CD32 are fused and combined with T-cell epitopes
- 1 small mutated immunoglobulin domain with 2 different specificities (TLR9 and CD32) is combined with T-cell epitopes

### Antigens and Epitopes

The antigens that are part of the molecule or molecule complex according to the invention can be complete allergens, denatured allergens or any antigens that are treated in any possible way to prevent binding to IgE. Such treatment may consist of epitope shielding of the antigenic protein using high affinity IgM, IgD, IgA or IgG antibodies directed to the same epitopes as the patient's IgE antibodies as described by Leroy et al [20]. Such antibodies may also bind close to the IgE specific epitopes thus preventing binding of the IgE antibodies by sterical hindrance.

Allergens are generally defined as antigens to which atopic patients respond with IgE antibody responses subsequently leading to allergic reactions. Antigens used in the molecule or the molecule complex of the invention can be environmental allergens (e.g. house dust mite, birch pollen, grass pollen, cat antigens, cockroach antigens), or food allergens (e.g. cow milk, peanut, shrimp, soya), or a combination of both. Also non relevant antigens such as HSA can be part of the molecule or molecule complex according to the invention. The antigen can be a complete allergen, exemplary an allergen for which patients with atopic dermatitis, allergic asthma, allergic rhinitis or allergic conjunctivitis are allergic. Preferably the allergen use in the molecule or molecule complex according ot the invention does not bind to IgE from the patient in need of treatment.

The antigens and/or epitopes used in the invention can be from natural sources or be produced by recombinant technology or be produced synthetically. Antigens and/or epitopes of the invention may contain ligand structures which facilitate incorporation of antigens and/or epitopes into molecule complexes of the invention via ligand/receptor interactions or antibody binding. Antigens and/or epitopes of the invention may contain chemical groups which facilitate covalent linkage of the antigens and/or epitopes to the CD32- and/or TLR9-binding structures of the molecule of the invention.

In one embodiment of the invention the antigens and epitopes of the molecule or molecule complex of the invention may be covalently linked to the CD32 binding structure and/or to the TLR9 binding structure.

In one embodiment antigens and/or epitopes may also be linked by a ligand/receptor interaction such as biotin and avidin to the molecule or molecule complex of the invention. For example, the antigens or epitopes to be used in the molecule of the invention may be produced with biotin or a biotin mimetic attached to it. The CD32 binding structure and/ or the TLR9 binding structure may be produced with avidin or another biotin-specific ligand attached to it. After mixing of these molecules with the different attachments, a stable molecule complex is formed according to the invention. Alternatively, an antibody/antigen binding can be used to form a molecule complex of the invention. High affinity interactions are preferred for these embodiments (e.g. high affinity anti-digoxigenin antibody and digoxigenin labeled antigens and/or epitopes).

In one embodiment of the invention the antigens and/or epitopes are genetically fused to the CD32-binding structure and/or to the TLR9-binding structure.

If the molecule of the invention is a fusion protein, the antigen is preferably produced from at least one T-cell epitope-containing DNA-subsequence of an allergen. The T cell epitopes can alternatively be from one or more related and/or unrelated allergens.

Preferably, the T cell epitopes comprise a new protein, which is not as such a naturally existing protein and therefore is not recognized by existing IgE or IgG antibodies in the patient. Therefore, instead of selecting short T cell epitopes which are cut apart and fused together again in a different order, one could also select a larger stretch of T cell epitopes (> 28 AA) which are still in their natural order but which have been previously selected not to bind to allergen specific IgE [21].

In principle all known antigens can be used for incorporation into the molecule or molecule complex of the invention to which allergic patients respond with IgE mediated hypersensitivity reactions. The most common environmental allergens in the developed countries are: House dust mite, birch pollen, grass pollen, cat, and cockroach. Each of these allergens has one or more "major allergens" (e.g. house dust mite: major allergen = Der P1; Der F1, birch pollen: major allergen = Bet V1). However, complete antigens, though possible, are not necessary, because the molecule or molecule complex should only induce T cell responses, and T cells respond to small (ca. 12-28 aminoacid long) peptides presented in MHC Class II molecules. The selection of T cell epitopes should be designed in such a way that expression on HLA class II molecules of possibly all patients is guaranteed. Some HLA class II molecules are more frequently expressed than others. A good example for such a HLA class II molecule with wide expression is HLA DPw4, which is expressed on approximately 78% of the Caucasian population [22]. Therefore a selection of T cell epitopes could be included in the molecule or molecule complex for each allergen, thus reducing the size and molecular weight of the complex. If overlapping cross-reactive epitopes between allergens from different genetically related organisms, such as Dermatophagoides pteronyssinus (Der P1) and Dermatophagoides farinae, (Der F1), are present, they are preferred.

To allow for correct antigen processing, DNA coding for stretches slightly longer then the actual T cell epitope should be included in the molecule or molecule complex and/or the epitopes can be separated from each other by introducing stretches of spacer DNA preferably containing (hydrophobic) epitopes recognized by major protein processing enzymes in antigen presenting cells such the asparagine-specific endopeptidase (AEP) or cathepsin S, cathepsin D or cathepsin L [23] .

For fusion to the genes coding for the binding structures specific for TLR9 and CD32, preferably short DNA sequences of major allergens are used such as house dust mite major allergen I (Der P1, Der F1 ), house dust mite major allergen II (Der P2, Der F2), or birch pollen allergen (Bet V1). These short DNA sequences contain the genetic code for one or more T cell epitopes, which after processing, appear on the surface of antigen presenting cells and therefore induce an immune response in the responding allergen specific T cells. Not only T cell epitopes from Der P1 and Der P2 but also Der P3, Der P4, Der P5, Der P6, Der P7 etc. and Der F3, Der F4, Der F5, Der F6, Der F7 etc can be used in a molecule or molecule complex of the invention. T cell epitopes from these allergens may be selected by classical epitope mapping using T cell clones [24] or by using modem HLA Class II predicting software such as the Tepitope program [25; 26]. For the molecule or molecule complexs, which can be formulated as vaccine, it is not necessary to combine T cell epitopes from a single allergen source only; to the contrary it is preferred to include as many T cell epitopes derived from different allergen sources produced by one or many different species, e.g a combination of allergens from house dust mites and of allergens from grass pollen, cats and/or birch pollen.

As an example for Der P1 the majority of the T cell epitopes can be found in the following sequences 101-143 of the mature protein in amino acid 1 letter code (SEQ ID No 4):

Especially the amino acid sequence 101-131 contains at least 3 T cell epitopes²⁴, which bind to a number of HLA class II molecules in amino acid 1 letter code (SEQ ID No 5):

The sequence 107-119 contains an important T cell epitope that binds to HLA DPw4 as well as HLA DPw5 ²⁴. These HLA Class II molecules are expressed by the majority of the population. The epitope in amino acid 1 letter code (SEQ ID No 6):

Other important T cell epitopes which in addition are shared between Der P1 and Der F1 are found in the sequences 20-44 and 203-226 of the mature protein in amino acid 1 letter code: and

Examples of T cells epitopes shared between Der P2 and Der F2 are found in the sequence 26-44, 89-107 and 102-123

From the above mentioned T cell epitopes of Der P1/F1 and Der P2/F2 one can design several functional molecule for molecule complexes, e.g:
By taking from Der P1 the following sequences: And from Der P 2

One can design a cDNA with the order B,A,E,H,G,C,F,D or H,A,D,C,F,G,E,B, but any possible combination of the selected sequences will do. The preferred order of the epitopes will largely be determined on the basis of expression efficiency of the complete recombinant molecule. Also duplications of sequences are allowed e.g. B,B,A,E,E,G,C,G,F,A,D etc. The T cell epitope part may of course also contain the genetic codes for shorter peptides or longer peptides for more and for fewer peptides, as long as one or more T cell epitopes from one or more different allergens/antigens are included.

Epitopes from other allergens such as Bet V1, Lol P1, Fel d1 with similar characteristics will be preferred for inclusion in the molecule or molecule complex according to the invention.

The invention also concerns a method of treating diseases, especially allergies, which comprises administering to a subject in need of such treatment a prophylactically or therapeutically effective amount of a molecule or molecule complex according to the invention for use as a pharmaceutical, especially as an agent against allergies.

The molecule or molecule complex may be admixed with conventional pharmaceutically acceptable diluents and carriers and, optionally, other excipients and administered parenterally intravenously or enterally, e.g. intramuscularly and subcutaneously. The concentrations of the molecule or molecule complex will, of course, vary depending i.a. on the compound employed, the treatment desired and the nature of the form.

For different indications the appropriate doses will, of course, vary depending upon, for example, the molecule or molecule complex used, the host, the mode of application and the intended indication. However, in general, satisfactory results are indicated to be obtained with 1 to 4 vaccinations in 1-2 years, but if necessary repeated additional vaccination can be done. It is indicated that for these treatments the molecule or molecule complex of the invention may be administered in 2-4 doses and with an application schedule similar as conventionally employed.

It further concerns a molecule or molecule complex according to the invention for use as a pharmaceutical, particularly for use in the treatment and prophylaxis of allergies.

The pharmaceutical composition prepared according to the present invention for use as vaccine formulation can (but does not have to) contain at least one adjuvant commonly used in the formulation of vaccines apart from the molecule or molecule complex. It is possible to enhance the immune response by such adjuvants. As examples of adjuvants, however not being limited to these, the following can be listed: aluminium hydroxide (Alu gel), QS-21, Enhanzyn, derivatives of lipopolysaccharides, Bacillus Calmette Guerin (BCG), liposome preparations, formulations with additional antigens against which the immune system has already produced a strong immune response, such as for example tetanus toxoid, Pseudomonas exotoxin, or constituents of influenza viruses, optionally in a liposome preparation, biological adjuvants such as Granulocyte Macrophage Stimulating Factor (GM-CSF), interleukin 2 (IL-2) or gamma interferon (IFNγ).
Aluminium hydroxide is the most preferred vaccine adjuvant.

Summary of a possible mode of action of the fusion protein according to the invention:

The fusion protein according to the present invention, can be formulated in any of the available acceptable pharmaceutical formulations, but is preferably formulated as a vaccine. The aCD32 binding portion of the fusion protein according to the invention selects the relevant cells. Triggering of CD32 on these cells will actively induce internalization of the receptor plus the attached fusion protein and by doing so facilitates the interaction of the TLR9 binding portion of the fusion protein with the TLR9, which is expressed within the cytoplasm of the relevant antigen presenting cells [10;11].

As a consequence of the CD32 mediated internalization, the subsequent processing and presentation of the selected T cell epitopes on MHC Class II molecules, combined with the specific activation of cytoplasmic TRL9 in the antigen presenting cells, allergen specific T cells will be (re-)programmed to become Th1 memory cells. These allergen specific Th1 memory cells at a later time point will induce allergen specific IgG production when encountering the same epitopes derived from the natural allergens presented by naturally exposed allergen specific B cells. These Th1 cells thus are necessary for rebalancing the immune system from IgE to IgG dominated antibody production.

### Examples:

The following examples shall explain the present invention in more detail, without, however, restricting it.

### Example 1:

Panning of the human CL - phage library on a TLR-9 peptide e.g. sequence 216-240 of the mature protein TLR9 (SEQ ID No 3) in amino acid 1 letter code

3 panning rounds shall be performed according to standard protocols. Briefly, the following method can be applied. Maxisorp 96-well plates (Nunc) are coated with the (synthetic) peptide representing part of the sequence of the TLR-9. For coating the peptides in the wells, 200 µl of the following solution are added per well: 0.1M Na-carbonate buffer, pH 9.6, with the following concentrations of dissolved peptide:
1st panning round: 1 mg/ml TLR-9 peptide
2nd panning round: 500 µg/ml TLR-9 peptide
3rd panning round: 100 µg/ml TLR-9 peptide
Incubation is for 1 hour at 37 °C, followed by blocking with 2% dry milk (M-PBS) with 200 µl per well for 1 hour at room temperature. The surface display phage library is then allowed to react with the bound peptide by adding 100 µl phage suspension and 100 µl 4% dry milk (M-PBS), followed by incubation for 45 minutes with shaking and for 90 minutes without shaking at room temperature. Unbound phage particles are washed away as follows. After the 1 st panning round: 10 x 300 µl T-PBS, 5x 300 µl PBS; after the 2nd panning round: 15 x 300 µl T-PBS, 10x 300 µl PBS; after the 3rd panning round: 20 x 300 µl T-PBS, 20 x 300 µl PBS. Elution of bound phage particles is performed by adding 200 µl per well of 0.1 M glycine, pH 2.2, and incubation with shaking for 30 minutes at room temperature. Subsequently, the phage suspension is neutralized by addition of 60 µl 2M Tris-Base, followed by infection into E. coli TG1 cells by mixing 10 ml exponentially growing culture with 0.5 ml eluted phage and incubation for 30 minutes at 37 °C. Finally, infected bacteria are plated on TYE medium with 1% glucose and 100 µg/ml Ampicillin, and incubated at 30 °C overnight.

### Example 2:

### Cloning of selected clones of human CL mutants selected against TLR-9 for soluble expression

Phagemid DNA from the phage selected through the 3 panning rounds is isolated with a midi-prep. DNA encoding mutated CL-regions is batch-amplified by PCR and cloned Ncol-Notl into the vector pNOTBAD/Myc-His, which is the E. coli expression vector pBAD/Myc-His (Invitrogen) with an inserted Notl restriction site to facilitate cloning. Ligated constructs are transformed into E. coli LMG194 cells (Invitrogen) with electroporation, and grown at 30 °C on TYE medium with 1% glucose and ampicillin overnight. Selected clones are inoculated into 200 µl 2xYT medium with ampicillin, grown overnight at 30 °C, and induced by adding L-arabinose to an end concentration of 0.1%. After expression at 16 °C overnight, the cells are harvested by centrifugation and treated with 100 µl Na-borate buffer, pH 8.0, at 4 °C overnight for preparation of periplasmic extracts. 50 µl of the periplasmic extracts were used in ELISA (see below).

### Example 3: ELISA of human CL mutants selected against TLR-9

selected clones are assayed for specific binding to the TLR-9 peptide by ELISA.
Coating: Microtiter plate (NUNC, Maxisorp), 100 µl per well, 20 µg TLR-9 peptide /ml 0.1 M Na-carbonate buffer, pH 9.6, 1 h at 37 °C
Wash: 3x 200 µl PBS
Blocking: 1% BSA-PBS, 1h at RT
Wash:3x 200 µl PBS
Periplasmic extract binding: 50 µl periplasmic extract
50 µl 2% BSA-PBS, at room temperature overnight
Wash: 3x 200 µl PBS
1st antibody: anti-His4 (Qiagen), 1:1000 in 1% BSA-PBS, 90 min at RT, 100 µl per well
Wash: 3x 200 µl PBS

2nd antibody: goat anti mouse*HRP (SIGMA), 1:1000 in 1% BSA-PBS, 90 min at RT, 100 µl per well
Wash: 3x 200 µl PBS
Detection: 3mg/ml OPD in Na-citrate/phosphate buffer, pH 4.5, 0.4 µl 30%H2O2
Stopping: 100 ml 3M H2SO4
Absorbance read: 492/620 nm

Clones that give a high signal in this first, preliminary ELISA are cultured in a 20-ml volume at the same conditions as described above. Their periplasmic extracts are isolated in 1/20 of the culture volume as described above and tested with ELISA (as described above) for confirmation.

### Example 4: Cloning of the anti-CD32 variable domains from HB-217

mRNA is isolated from the cell line HB-217 (ATCC, antiCD32 antibody IV.3) and is used to prepare cDNA according to established routine protocols. The cDNA is further used as a template to amplify the regions of the genes coding for the of the light and the heavy chain of the Fab fragment of antibody IV.3 respectively. Upstream PCR primers, which prime from the 5' end of the variable regions, used for this amplification are derived from the published sequences of mouse variable regions (IMGT, the international ImMunoGeneTics information system® http://imgt.cines.fr). Degenerate primers and/or mixtures of different primers are used as upstream primers. Downstream primers are designed such as to prime from the 3' end of the CL or the CH1 domains respectively.

In a next step, the CL domain of the antibody IV.3 is removed and replaced by a selected CL domain modified by SMID technology which has binding affinity to TLR9, and which is selected as described above in examples 1-3. For this replacement, overlapping PCR can be used according to standard protocols. Alternatively, for joining VL to the SMID modified CL a uniquely cutting restriction site can be used which is either naturally occurring in the sequence or which is artificially introduced by site directed mutagenesis (as a silent mutation which does not change the amino acid sequence). For example, a BstAPI site can be generated in the hinge region between VL and CL by changing the sequence from:
K R A D A A P T V S I F (SEQ ID No 65)
AAACGGGCTGATGCTGCACCAACTGTATCCATCTTC (SEQ ID No 66)
   to:
K R A D A A P T V S I F (SEQ ID No 65)
AAACGG*GCAGATGCTGC*ACCAACTGTATCCATCTTC (SEQ ID No 15)
the newly created BstAPI site is highlighted in the above sequence. The new sequence is introduced in the coding regions by amplifying the VL part and the CL part respectively with appropriately designed PCR primers, cutting the PCR products with BstAPI, ligating them, and amplifying the complete resulting ligation product with PCR primers as used initially for amplifying the original light chain part of the Fab fragment.

For expression of the modified Fab fragment, the genes coding for the heavy and the light chains are subsequently cloned in appropriate expression vectors, or together in one expression vector which allows the expression of two independent genes. As an expression system, bacteria, yeast, animal cells or any other suitable expression system can be used. For this example here, expression from one vector in the methylotrophic yeast *Pichia pastoris* will be shown:

The light chain part of the modified PCR fragment is cloned EcoRI / Kpnl in the Pichia pastoris expression vector pPICZalphaA in the correct reading frame such as to fuse it functionally with the alpha-factor secretion signal sequence provided by the vector. Similarly, the heavy chain part of the Fab fragment is cloned in pPICZalphaA. In order to prepare the inserts for this cloning procedure, appropriately designed PCR primers are used which attach the needed restriction sites to the genes. At the 3' ends of both coding regions, a stop codon has to be inserted and provided by the PCR primers as well. The light chain expression cassette is then cut out from the vector with restriction enzymes BgIII and BamHI, and the ends of the DNA are made blunt by treatment with Klenow fragment of DNA polymerase. The vector containing the inserted heavy chain part of the Fab is opened by a partial digest with restriction enzyme BgIII, the DNA is made blunt by treatment with Klenow fragment of DNA polymerase, and the expression cassette coding for the light chain part is inserted. The partial digest of the heavy chain vector is necessary since the inserted heavy chain gene contains a BgIII site. For screening of the final construct, care has to be taken that this internal BgIII site has remained intact. The final construct has one Pmel site which is used for linearizing the construct prior to transformation into *Pichia pastoris.* This linearization is advantageous for efficient integration of the expression vector in the host genome by homologous recombination. *Pichia pastoris* is transformed with the linearized expression vector using electroporation, transformed clones are selected with the antibiotic Zeocin for which the vector confers resistance, and supernatants of randomly picked clones are screened for expression of the Fab construct after induction of expression with methanol. For screening, e.g. a Fab-specific ELISA can be used. Production of the recombinant protein is achieved by culturing the transformed selected Pichia clone in a larger scale, preferable in shake flasks or in a fermenter, inducing expression by addition of methanol and purifying the recombinant protein by a chromatographic method. For these latter steps, routine protocols are used.

### Example 5: Cloning of the Der P1I/F1 and Der P2/F2 derived T cell epitopes:

The combination of the selected T cell epitopes formed by sequences B,A,E,H,G,C,F,D looks a follows (SEQ ID No 16):

In order to construct a synthetic gene coding for this amino acid sequence, in silico reverse translation can be used. Computer programs are available for this purpose, such as e.g. DNAWORKS (hftp://molbio.info.nih.gov/dnaworks/). In order to clone the synthetic gene coding for the epitopes in frame with the gene coding for the heavy chain part of the framework, two restriction sites are selected which cut neither on this coding region nor on the vector pPICZalphaA. For example, AccIII and Spel can be used for this purpose. These two restriction sites are attached to the gene coding for the heavy chain part of the Fab by using appropriately designed PCR primers for the cloning procedure as described above. Furthermore, care has to be taken not to have a stop codon at the end of the coding region of the heavy chain part of the Fab, as the stop codon will be provided at the 3' end of the synthetic gene coding for the epitopes. Again, this construct with the two additional restriction sites located at its 3' end is cloned EcoRI / Kpnl in the *Pichia pastoris* expression vector pPICZalphaA. The construct is then opened with the restriction enzymes AccIII and Spel and the insert coding the epitopes in inserted. This insert is generated as follows:

The chosen amino acid sequence together with the chosen restriction sites, in this example AccIII at the 5' end and Spel at the 3' end are used as input in the publicly available computer program DNAWORKS. In addition, a stop codon is added between the end of the epitope sequence and the Spel site.

The parameters which the program uses for designing the oligonucleotides are left at the proposed standard values, and the program is instructed to avoid the sequences of the restriction sites which are necessary for the cloning and transformation steps, such as AccIII, Spel and Pmel.
AccIII: tccgga
Spel: actagt
Pmel: gtttaaac
DNAWORKS generates a set of oligonucleotides which are overlapping and which represent both strands of the desired coding regions.

For example, the following set of 24 oligonucleotides is generated, from which the synthetic gene coding for the allergen epitopes is generated:

| | | | |
|---|---|---|---|
| 1 | TCCGGATGCCAAATTTACCCGCCAAACG | 28 | **(SEQ ID No 17)** |
| 2 | AGCCTCTCTGATCTTGTTCGCGTTTGGCGGGTAAATTTGG | 40 | **(SEQ ID No 18)** |
| 3 | CGAACAAGATCAGAGAGGCTTTGCAATCTTGCAGGAGGCC | 40 | **(SEQ ID No 19)** |
| 4 | TATGCCGAATCTCTGCGCATTGGGCCTCCTGCAAGATTGC | 40 | **(SEQ ID No 20)** |
| 5 | GCGCAGAGATTCGGCATATCCAACTACTGCCAGATCTACC | 40 | **(SEQ ID No 21)** |
| 6 | GTACGCCCATCGTATGGGGGGTAGATCTGGCAGTAGTTGG | 40 | **(SEQ ID No 22)** |
| 7 | CCCATACGATGGGCGTACAATCATACAGCGTGATAACGGC | 40 | **(SEQ ID No 23)** |
| 8 | GCGTGGTAGTTAGGCTGATAGCCGTTATCACGCTGTATGA | 40 | **(SEQ ID No 24)** |
| 9 | TATCAGCCTAACTACCACGCCGTGAACATCGTCGGCTACG | 40 | **(SEQ ID No 25)** |
| 10 | TCACAGTAACCACGACATTCTCGTAGCCGACGATGTTCAC | 40 | **(SEQ ID No 26)** |
| 11 | AGAATGTCGTGGTTACTGTGAAGGTAATGGGCGATGACGG | 40 | **(SEQ ID No 27)** |
| 12 | AGCTATGGCGCAAGCTAGAACCCCGTCATCGCCCATTACC | 40 | **(SEQ ID No 28)** |
| 13 | TCTAGCTTGCGCCATAGCTACCAAGTACACTTGGAACGTA | 40 | **(SEQ ID No 29)** |
| 14 | TTTTCGGCGCAATTTTGGGTACGTTCCAAGTGTACTTGGT | 40 | **(SEQ ID No 30)** |
| 15 | CCCAAAATTGCGCCGAAAAGTGAAAACGTCGTAGTGACCA | 40 | **(SEQ ID No 31)** |
| 16 | TGAGCCAATGCCTCCCTTATGGTCACTACGACGTTTTCAC | 40 | **(SEQ ID No 32)** |
| 17 | AGGGAGGCATTGGCTCAACCTCAAAGATACTGCAGACACT | 40 | **(SEQ ID No 33)** |
| 18 | TTATGCAGGGCGTCCAGTAGTGTCTGCAGTATCTTTGAGG | 40 | **(SEQ ID No 34)** |
| 19 | ACTGGACGCCCTGCATAATCCACCGTGGTAAACCCTTTCA | 40 | **(SEQ ID No 35)** |
| 20 | CTTCGAACACTGCCTCAAGTTGAAAGGGTTTACCACGGTG | 40 | **(SEQ ID No 36)** |
| 21 | ACTTGAGGCAGTGTTCGAAGCTAACAGGACGGTAACGCCA | 40 | **(SEQ ID No 37)** |
| 22 | CCGCACCCACCTTGCATACGAATTGGCGTTACCGTCCTGT | 40 | **(SEQ ID No 38)** |
| 23 | TGCAAGGTGGGTGCGGGTCTTGTTGGGCTTTTTCTGGTGT | 40 | **(SEQ ID No 39)** |
| 24 | ACTAGTTTATTCAGTAGCAGCCACACCAGAAAAAGCCCAACA | 42 | **(SEQ ID No 40)** |

These 24 oligonucleotides are dissolved, mixed together, boiled for several minutes and then cooled down to room temperature slowly to allow annealing. In a subsequent PCR steps using large amounts of the two bordering primers (primers #1 and #24), the annealed gene is amplified, the PCR product is then cleaved with the chosen restriction enzymes (AccIII and Spel in this example), and cloned into the expression vector as described above, which contains as an insert the gene coding for the heavy chain part of the modified Fab. Preparation of the final expression vector containing both chains, transformation of *Pichia pasforis*, selection of clones and screening for producing clones is done as described above. Expression and purification of the recombinant protein is performed by following standard protocols.

### Example 6

### Fusion of VH and VL of the anti-CD32 antibody IV.3 fusion with anti-TLR9 CH3 domains (SMIDS)

All molecular modeling was done with Swiss-PdbViewer 3.7 (http://swissmodel.expasy.org/spdbv/)

As a homology model for a mouse Fab fragment, the structure file 2BRR.pdb from the Protein Data Bank (www.pdb.org) is used, and 10QO.pdb is used as a source for the structure of a human IgG CH3 domain.

Molecular models of VH and VL of the IV.3 antibody are made with the "first approach mode" of Swissmodel http://swissmodel.expasy.org/SWISS-MODEL.html) using the amino acid sequences of VH and VL respectively.

Using the "magic fit" function of the Swiss-PdbViewer, two copies of the CH3 domain structure from 10QO.pdb are fitted onto the CH1 and the CL domain respectively of 2BRR.pdb. Subsequently, the molecular models of the IV.3 VH and VL respectively are fitted (again using "magic fit") onto VH and VL of 2BRR.pdb.

For construction of an Fab-like protein in which CH1 and CL are both replaced by a CH3 domain, it is necessary to decide at which point the sequence of VH should be ended and connected to the sequence of CH3, and at which point the sequence of VL should be ended and connected to the sequence of CH3. For both constructs, a point is chosen at which the main chain of the superimposed structures and models (see above) shows an optimal overlap.

For the light chain, it was found that the sequence up to Ala114 (numbering from 2BRR.pdb) will be used an connected to Pro343 (numbering from 1OQO.pdb) of the CH3 domain. The point of connection between these two sequences therefore reads as follows (VL part is underlined):
- - -Lys112 - Arg113-Ala114-Pro343-Acg344-Glu345 - -

In order to allow joining of the two coding sequences using restriction enzyme sites and DNA ligation, the sequence near the point of connection is changed by silent mutation to introduce a unique Xhol site (ctcgag, undertined) as follows:
K R A P R E (SEQ ID No 41)
AAACGGGCT**CCTCGAGAA** (SEQ ID No 42)

For later insertion of the allergen epitopes, an AscI site (ggcgcgcc) is introduced just before the stop codon of the construct plus an extra base for maintenance of the reading frame:
ggg cgc gcc
Gly Arg Ala

Furthermore, for cloning into the expression vector pPlCZalphaA (Pichia pastoris expression system, Invitrogen), an EcoRI site (gaattc) is added to the 5'-end (N-terminus) and a KpnI site (ggtacc) to the 3'-end (C-terminus) of the construct.

The CH3 domain to be fused to VH and VL respectively selected as part of the construct can be a wildtype human IgG CH3 domain which can serve as a negative control, or a CH3 domain previously engineered by SMID technology and selected to bind specifically to TLR9. In this example here, the sequence of clone A23, which binds specificall to TLR9 and which was described in the patent application PCT/EP2006/050059 is fused to both, VH and VL.

Therefore, the complete sequence of the VL-CH3 fusion protein has the following amino acid sequence (VL part is underlined), (SEQ ID No 43):

Nucleic acid sequence of the VL-CH3 fusion protein (restriction sites are underlined), (SEQ ID No 44):

For the heavy chain, it was found that the sequence up to Thr123 (numbering from 2BRR.pdb) should be used an connected to Arg344 (numbering from 1OQO.pdb) of the CH3 domain. The point of connection between these two sequences therefore reads as follows (VH part is underlined):
- - - Ala121 - Lys122 - Thr123 - Arg344 - Glu345 - Pro346 - - -

In order to allow joining of the two coding sequences using restriction enzyme sites and DNA ligation, the sequence near the point of connection was changed by silent mutation to introduce a unique Xhol site (ctcgag, underlined) as follows:
A K T R E P (SEQ ID No 45)
GCCAAAACTCGAGAACCA (SEQ ID No 46)

Furthermore, for cloning into the expression vector pPICZalphaA (Pichia pastoris expression system, Invitrogen), an EcoRI site (gaattc) is added to the 5'-end (N-terminus) and an Xbal site (tctaga) to the 3'-end (C-terminus) of the construct. No stop codon is added to this sequence and the Xbal site is placed in the correct reading frame so as to fuse the construct to the Hexa-His-tag provided by the vector for later purification of the protein using immobilized metal affinity chromatography.

Therefore, the complete sequence of the VH-CH3 fusion protein has the following amino acid sequence (VH part is undermined), (SEQ ID No 47):

Nucleic acid sequence of the VH-CH3 fusion protein (restriction sites are underlined), (SEQ ID No 48):

### Detailed cloning plan

### Heavy chain:

The VH region of antibody IV.3 is PCR-amplified with primers 4.3HupEco and 4.3HdownXho, and subsequently digested with EcoRI and Xhol. The CH3 SMID-engineered clone A23 is PCR-amplified with primers CH3upXhoA and CH3XBA2 and subsequently digested with Xhol and Xbal. The VH sequence and the CH3 sequence are ligated together via the XhoI site and then ligated into pPICZalphaA (Invitrogen), which was previously digested with EcoRI and Xbal. The resulting vector is named pPICHA23.

### PRIMER LIST :

| | |
|---|---|
| 4.3HUPECO | cagagaattc gaggttcagc ttcagcagtc **(SEQ ID No 49)** |
| 4.3HDOWNXHO | gatgctcgag ttttggctga ggagacggtg **(SEQ ID No 50)** |
| CH3UPXHOA | aaaactcgag aaccacaggt gtacaccctg cc **(SEQ ID No 51)** |
| CH3XBA2 | actgatctag acctttaccc ggagacaggg agag **(SEQ ID No 52)** |

### Light chain:

The VL region of antibody IV.3 is PCR-amplified with primers 4.3LupEco and 4.3LdownXho, and subsequently digested with EcoRI and Xhol. The CH3 SMID-engineered clone A23 is PCR-amplified with primers CH3upXhoB and CH3StopKpn and subsequently digested with Xhol and Kpnl. The VL sequence and the CH3 sequence are ligated together via the Xhol site and then ligated into pPICZalphaA (Invitrogen), which was previously digested with EcoRI and Kpnl. The resulting vector is named pPICLA23.

### PRIMER LIST :

| | |
|---|---|
| 4.3LUPECO | gatagaattc gacattgtga tgacccaggc tg **(SEQ ID No 53)** |
| 4.3LDOWNXHO | attactcgag gagcccgttt cagttccagc t **(SEQ ID No 54)** |
| CH3UPXHOB | gctcctcgag aaccacaggt gtacaccctg cc **(SEQ ID No 55)** |
| CH3STOPKPN | acgtggtacc tcaggcgcgc cctttacccg gagacaggga gag **(SEQ ID No 56)** |

### COMBINATION OF THE TWO EXPRESSION CASSETTES IN ONE VECTOR

The light chain cassette is cut out with BgIII (pos.1) and BamHI (pos. 2319) from pPICLA23 (4235 bp), and the 2319 bp fragment is purified via preparative gele electrophoresis. The 1916 bp fragment is discarded. The vector pPICHA23 (4219 bp) is digested with BamHI, and the previously purified 2319 bp fragment from pPICLA23 is inserted. The resulting Pichia pastoris expression vector, which carries two expression cassettes, one for the VL-CH3 fusion protein and on for the VH-CH3 fusion protein is screened so that both inserts that have same direction of transcription. The resulting vector pPICHLA23 (6537 bp) is then linearized before transformation into Pichia pastoris e.g. with BamHI or with BssSI, transformed into Pichia pastoris by electroporation, and positive transformants are selected with Zeocin. Several clones are screened for expression of the recombinant protein. A clone is then selected for large scale production, and the recombinant fusion protein is purified by immobilized-metal-affinity chromatography using standard procedures. All Pichia manipulation, culturing and expression is done by following standard protocols (Invitrogen).

### Insertion of allergen epitopes into the vector pPICHLA23 and expression of the recombinant fusion protein

The sequence encoding the allergen epitopes as described in example 5 is inserted into the vector pPICHLA23 as follows:

The vector is digested with AscI (4174-4182) which leads to its linearization. In this Ascl site, the DNA sequence encoding the allergen epitopes is inserted. The sequence encoding the allergen epitopes is amplified with primers EpiTLR1 and EpiTLR2 in order to attach AscI sites to both ends of the sequence.
Primer list
EpiTLR1 TAAAGGGCGC GCCTCCGGAT GCCAAATTTA CC
   (SEQ ID No 57)
EpiTLR2 TACCTCAGGC GCGCCTTATT CAGTAGCAGC CACAC
   (SEQ ID No 58)

The resulting PCR product is digested with AscI and ligated into the previously digested vector. The resulting vector is named pHLA23EP (7046 bp). Pichia transformation, expression and purification of the recombinant fusion protein is performed as described above for the construct that has no epitopes inserted.

### VL of antibody IV.3:

### amino acid sequence:

### nucleic acid sequence:

### VH of antibody IV.3:

### amino acid sequence:

### nucleic acid sequence:

### Final expression vector pPICHCLA23.seq. (SEQ ID No 63) containg TLR9 and CD32 binding regions

### 6537 bp

### Final expression vector pHLA23EP.seq (SEQ ID No 64) containg TLR9 and CD32 binding regions and epitope sequence (see SEQ ID No 16)

All temperatures are in degrees Celsius. The following abbreviations are used: CD32 = FcγRII
TLR9 = Toll like receptor 9
Der P1 = Dermatophagoides pteronissyus major allergen 1
Der P2 = Dermatophagoides pteronissyus major allergen 2
Der F1 = Dermatophagoides farinae major allergen 1

In the following, preferred aspects of the present disclosure are disclosed:
1. A molecule or molecule complex capable of binding to TLR9 and to CD32 comprising at least one epitope of at least one antigen.
2. A molecule or molecule complex according to 1., characterized in that the epitope is a T-cell epitope
3. A molecule or molecule complex according to any one of 1. or 2., characterized in that the epitope is derived from an allergen.
4. A molecule or molecule complex according to any one of 1. to 3., characterized in that at least one epitope is non-covalently linked to the molecule.
5. A molecule or molecule complex according to any one of 1. to 4., characterized in that at least one epitope is non-covalently linked to the TLR9 and/or CD32 binding region.
6. A molecule or molecule complex according to 5., characterized in that at least one epitope is linked to the TLR9 and/or CD32 binding region via antibody interaction and/or ligand interaction.
7. A molecule or molecule complex according to any one of 1 to 6., characterized in that the epitope is produced from at least one T-cell epitope-containing-DNA-stretch of an antigen or a synthetic peptide
8. A molecule or molecule complex according to any one of 1. to 7., characterized in that the epitope is selected from the group consisting of allergens in atopic dermatitis, allergic asthma, allergic rhinitis or allergic conjunctivitis.
9. A molecule or molecule complex according to any one of 1. to 8., characterized in that the epitope is isolated from complete antigens, denatured antigens or antigens modified to prevent binding to IgE.
10. A molecule or molecule complex according to any one of 1 to 9., characterized in that it comprises at least one antibody or derivative or fragment thereof
11. A molecule or molecule complex according to 10 , characterized in that the antibody or fragment or derivative thereof is IgG, IgM, IgE, IgA or IgD
12. A molecule or molecule complex according to any one of 1. to 11., characterized in that it is of human or humanized structure
13. A molecule or molecule complex according to any one of 1. to 12., characterized in that it is of murine or partly murine structure
14. A molecule or molecule complex according to any one of 1 to 13., charactenzed in that it is of camel or partly camel structure
15. A molecule or molecule complex according to any one of 1 to 14., characterized in that it comprises at least one engineered binding scaffold
16. A molecule or molecule complex according to 15., characterized in that the binding scaffold is selected from the group consisting of fibronectin III, lipocalins, Protein A, α-amylase inhibitor, Ankyrin Repeat Proteins, a C2 domain, an A-domain, an EGFR like domain, a dab, a chi-bAb, CTLA-4, gamma crystalline and any other protein
17. A molecule or molecule complex according to any one of 1 to 16., characterized in that it comprises at least one part of a small mutated immunoglobulin domain (SMID).
18. A molecule or molecule complex according to any one of 1 to 17., characterized in that it comprises at least part of an anti-CD32 antibody or derivative or fragment thereof.
19. A molecule or molecule complex according to any one of 1. to 18 comprising an amino acid sequence of SEQ ID No 59 or part thereof
20. A molecule or molecule complex according to any one of 1 to 19 comprising an amino acid sequence of SEQ ID No 61 or part thereof
21. Pharmaceutical composition comprising at least one molecule or molecule complex according to any one of 1 to 20 optionally together with at least one pharmaceutically acceptable carrier or diluent
22. Use of a pharmaceutical preparation according to 21 for active immunotherapy.
23. Method of treating allergies wherein a prophylactically or therapeutically effective amount of at least one molecule or molecule complex according to any one of 1 to 20 is administered to a subject in need of such treatment optionally together with at least one pharmaceutically acceptable carrier.
24. Use of at least one molecule or molecule complex according to any one of 1 to 20 for the manufacture of a medicament for the treatment of allergies.
25. Process for producing a molecule or molecule complex according to any one of 1 to 20., using recombinant technique wherein the genes coding for the binding structures for TLR9, CD32 and the epitope are constructed in a vector and expressed in a host cell
26. Nucleic acid vector for expressing of a molecule according to any of 1. to 20. comprising the nucleic acid sequence of SEQ ID No 60.
27. Nucleic acid vector for expressing of a molecule according to any of 1. to 20. comprising the nucleic acid sequence of SEQ ID No 62.
28. Nucleic acid vector for expressing of a molecule according to any of 1. to 20. comprising the nucleic acid sequence of SEQ ID No 63.
29. Nucleic acid vector for expressing of a molecule according to any of 1. to 20. comprising the nucleic acid sequence of SEQ ID No 64.
30. Process for producing a molecule or molecule complex according to any one of 1. to 20., using chemical cross linking.

### References

1. Mudde,G.C., I.G.Reischl, N.Corvaïa, A.Hren, and E.-M.Pöllabauer. 1996. Antigen presentation in allergic sensitization. Immunol. Cell Biol. 74:167-173.
2. Bheekha Escura,R., E.Wasserbauer, F.Hammerschmid, A.Pearce, P.Kidd, and G.C.Mudde. 1995. Regulation and targetting of T-cell immune responses by IgE and IgG antibodies. Immunology 86:343-350.
3. Pène,J., F.Rousset, F.Brière, I.Chrétien, J.-Y.Bonnefoy, H.Spits, T.Yokota, K.-I.Arai, J.Banchereau, and J.E.De Vries. 1988. IgE production by normal human lymphocytes is induced by interleukin 4 and suppressed by interferons gamma and alpha and prostaglandin E2. Proc. Natl. Acad. Sci. USA 85:6880-6884.
4. Ebner,C. 1999. Immunological mechanisms operative in allergen-specific immunotherapy. Int Arch Allergy Immunol 119:1-5.
5. Ferreira,F., C.Ebner, B.Kramer, G.Casari, P.Briza, A.J.Kungl, R.Grimm, B.Jahn-Schmid, H.Breiteneder, D.Kraft, M.Breitenbach, H.J.Rheinberger, and O.Scheiner. 1998. Modulation of IgE reactivity of allergens by site-directed mutagenesis: potential use of hypoallergenic variants for immunotherapy. FASEB Journal 12:231-242.
6. Rissoan,M.C., V.Soumelis, N.Kadowaki, G.Grouard, F.Briere, R.D.Malefyt, and Y.J.Liu. 1999. Reciprocal control of T helper cell and dendritic cell differentiation. Science 283:1183-1186.
7. Kapsenberg,M.L., C.M.Hilkens, E.A.Wierenga, and P.Kalinski. 1999. The paradigm of type 1 and type 2 antigen-presenting cells. Implications for atopic allergy. Clin. Exp. Allergy 29:33-36.
8. Charbonnier,A.S., H.Hammad, P.Gosset, G.A.Stewart, S.Alkan, A.B.Tonnel, and J.Pestel. 2003. Der p1-pulsed myeloid and plasmacytoid dendritic cells from house dust mite-sensitized allergic patients dysregulate the T cell response. J. Leukocyte Biol. 73:91-99.
9. Rothenfusser,S., E.Tuma, S.Endres, and G.Hartmann. 2002. Plasmacytoid dendritic cells: The key to CpG. Hum. Immunol. 63:1111-1119.
10. Latz,E., A.Schoenemeyer, A.Visintin, K.A.Fitzgerald, B.G.Monks, C.F.Knetter, E.Lien, N.J.Nilsen, T.Espevik, and D.T.Golenbock. 2004. TLR9 signals after translocating from the ER to CpG DNA in the lysosome. Nat. Immunol. 5:190-198.
11. Leifer,C.A., M.N.Kennedy, A.Mazzoni, C.W.Lee, M.J.Kruhlak, and D.A.Segal. 2004. TLR9 Is localized in the endoplasmic reticulum prior to stimulation. J. Immunol. 173:1179-1183.
12. Wang,W.W., D.Das, X.L.Tang, W.Budzynski, and M.R.Suresh. 2005. Antigen targeting to dendritic cells with bispecific antibodies. J. Immunol. Methods*.*
13. van Schaijk,F.G., E.Oosterwijk, A.C.Soede, M.Broekema, C.Frielink, W.J.McBride, D.M.Goldenberg, F.H.Corstens, and O.C.Boerman. 2005. Pretargeting of carcinoembryonic antigen-expressing tumors with a biologically produced bispecific anticarcinoembryonic antigen x anti-indium-labeled diethylenetriaminepentaacetic acid antibody. Clin. Cancer Res. 11:7130s-7136s.
14. Le,G.F., U.Reusch, G.Moldenhauer, M.Little, and S.M.Kipriyanov. 2004. Immunosuppressive properties of anti-CD3 single-chain Fv and diabody. J. Immunol. Methods 285:111-127.
15. Schuster,M., P.Umana, C.Ferrara, P.Brunker, C.Gerdes, G.Waxenecker, S.Wiederkum, C.Schwager, H.Loibner, G.Himmler, and G.C.Mudde. 2005. Improved effector functions of a therapeutic monoclonal Lewis Y-specfic antibody by glycoform engineering. Cancer Res 65:7934-7941.
16. Orlandi,R., D.H.Gussow, P.T.Jones, and G.Winter. 1989. Cloning immunoglobulin variable domains for expression by the polymerase chain reaction. Proc. Natl. Acad. Sci. U. S. A. 86:3833-3837.
17. Barton,G.M., J.C.Kagan, and R.Medzhitov. 2005. Intracellular localization of Toll-like receptor 9 prevents recognition of self DNA but facilitates access to viral DNA. Nat. Immunol*. ..*
18. Chen,W.L., J.L.Wang, H.Z.An, J.Zhou, L.H.Zhang, and X.T.Cao. 2005. Heat shock up-regulates TLR9 expression in human B cells through activation of ERK and NF-kappaB signal pathways. Immunol. Lett. 98:153-159.
19. Eaton-Bassiri,A., S.B.Dillon, M.Cunningham, M.A.Rycyzyn, J.Mills, R.T.Sarisky, and M.L.Mbow. 2004. Toll-like receptor 9 can be expressed at the cell surface of distinct populations of tonsils and human peripheral blood mononuclear cells. Infect. Immun. 72:7202-721.
20. Leroy,B.P., J.-M.Lachapelle, M.Jacquemin, and J.-M.Saint-Remy. 1992. Treatment of atopic dermatitis by allergen-antibody complexes: Long-term clinical results and evolution of IgE antibodies. Dermatologica 184:271-274.
21. Chua,K.Y., W.K.Greene, P.Kehal, and W.R.Thomas. 1991. IgE binding studies with large peptides expressed from Der p II cDNA constructs. Clin. Exp. Allergy. 21:161-166.
22. Baselmans,P.J., E.Pollabauer, F.C.Van Reijsen, H.C.Heystek, A.Hren, P.Stumptner, M.G.Tilanus, W.C.Vooijs, and G.C.Mudde. 2000. IgE production after antigen-specific and cognate activation of HLA- DPw4-restricted T-cell clones, by 78% of randomly selected B-cell donors. Hum. Immunol. 61:789-798.
23. Beck,H., G.Schwarz, C.J.Schroter, M.Deeg, D.Baier, S.Stevanovic, E.Weber, C.Driessen, and H.Kalbacher. 2001. Cathepsin S and an asparagine-specific endoprotease dominate the proteolytic processing of human myelin basic protein in vitro. Eur J Immunol 31:3726-3736.
24. Higgins,J.A., C.J.Thorpe, J.D.Hayball, R.E.O'Hehir, and J.R.Lamb. 1994. Overlapping T-cell epitopes in the group I allergen of Dermatophagoides species restricted by HLA-DP and HLA-DR class II molecules. J. Allergy Clin. Immunol. 93:891-899.
25. Bian,H., J.F.Reidhaar-Olson, and J.Hammer. 2003. The use of bioinformatics for identifying class II-restricted T-cell epitopes. Methods 29:299-309.
26. Sturniolo,T., E.Bono, J.Ding, L.Raddrizzani, O.Tuereci, U.Sahin, M.Braxenthaler, F.Gallazzi, M.P.Protti, F.Sinigaglia, and J.Hammer. 1999. Generation of tissue-specific and promiscuous HLA ligand databases using DNA microarrays and virtual HLA class II matrices. Nat Biotechnol. 17:555-561.

## Claims

1. A pharmaceutical preparation comprising a molecule or molecule complex capable of binding to TLR9 and to CD32, comprising at least one epitope of at least one antigen, wherein the CD32 binding part of the molecule or molecule complex is selected from the group consisting of antibodies, antibody fragments, protein domains and peptides, and the TLR9 binding part is a nucleic acid..

2. The pharmaceutical preparation according to claim 1, **characterized in that** the epitope is a T-cell epitope.

3. The pharmaceutical preparation according to claim 1 or 2, **characterized in that** the epitope is derived or produced from at least one T-cell epitope - containing - DNA-stretch of an antigen or a synthetic peptide.

4. The pharmaceutical preparation according to any one of claims 1 to 3, **characterized in that** the epitope is selected from the group consisting of allergens, specifically allergens in atopic dermatitis, allergic asthma, allergic rhinitis or allergic conjunctivitis.

5. The pharmaceutical preparation according to any one of claims 1 to 4, **characterized in that** the epitope is isolated from complete antigens, denatured antigens or antigens modified to prevent binding to IgE.

6. The pharmaceutical preparation according to any one of claims 1 to 5, **characterized in that** it comprises at least one antibody or derivative or fragment thereof.

7. The pharmaceutical preparation according to any one of claims 1 to 6 **characterized in that** the CD32 binding part is at least part of an anti-CD32 antibody or derivative or fragment thereof.

8. The pharmaceutical composition according to any one of claims 1 to 7, for use in active immunotherapy.

9. The pharmaceutical composition for use according to claim 8, for the treatment or prophylaxis of allergies.

10. Process for producing a molecule or molecule complex according to any one of claims 1 to 7, using recombinant technique, wherein the genes coding for the binding structures for TLR9, CD32 and the epitope are constructed in a vector and expressed in a host cell.

11. Process for producing a molecule or molecule complex according to any one of claims 1 to 7, using chemical cross linking.

## Patentansprüche

1. Pharmazeutische Zubereitung, umfassend ein Molekül oder einen Molekülkomplex, das bzw. der zum Binden an TLR9 und CD32 in der Lage ist, umfassend mindestens ein Epitop mindestens eines Antigens, wobei der CD32-bindende Teil des Moleküls oder Molekülkomplexes aus der Gruppe ausgewählt wird, die aus Antikörpern, Antikörperfragmenten, Proteindomänen und Peptiden besteht, und es sich bei dem TLR9-bindenden Teil um eine Nukleinsäure handelt.

2. Pharmazeutische Zubereitung nach Anspruch 1, **dadurch gekennzeichnet, dass** es sich bei dem Epitop um ein T-Zell-Epitop handelt.

3. Pharmazeutische Zubereitung nach Anspruch 1 oder 2, **dadurch gekennzeichnet, dass** das Epitop von mindestens einem T-Zell-Epitop-enthaltenden DNA-Abschnitt eines Antigens oder eines synthetischen Peptids abstammt oder aus einem solchen hergestellt wird.

4. Pharmazeutische Zubereitung nach einem der Ansprüche 1 bis 3, **dadurch gekennzeichnet, dass** das Epitop aus der Gruppe ausgewählt ist, die aus Allergenen besteht, insbesondere aus Allergenen bei atopischer Dermatitis, allergischem Asthma, allergischer Rhinitis oder allergischer Konjunktivitis.

5. Pharmazeutische Zubereitung nach einem der Ansprüche 1 bis 4, **dadurch gekennzeichnet, dass** das Epitop aus vollständigen Antigenen, denaturierten Antigenen oder Antigenen, die modifiziert sind, um die Bindung an IgE zu verhindern, isoliert ist.

6. Pharmazeutische Zubereitung nach einem der Ansprüche 1 bis 5, **dadurch gekennzeichnet, dass** sie mindestens einen Antikörper oder ein Derivat oder ein Fragment davon umfasst.

7. Pharmazeutische Zubereitung nach einem der Ansprüche 1 bis 6, **dadurch gekennzeichnet, dass** der CD32-bindende Teil mindestens Teil eines Anti-CD32-Antikörpers oder eines Derivats oder Fragments davon ist.

8. Pharmazeutische Zusammensetzung nach einem der Ansprüche 1 bis 7 zur Verwendung bei einer aktiven Immuntherapie.

9. Pharmazeutische Zusammensetzung zur Verwendung nach Anspruch 8 für die Behandlung von oder Vorbeugung gegen Allergien.

10. Prozess zum Herstellen eines Moleküls oder Molekülkomplexes nach einem der Ansprüche 1 bis 7 mithilfe einer Rekombinationstechnik, wobei die Gene, welche die Bindungsstrukturen für TLR9, CD32 und das Epitop kodieren, in einem Vektor konstruiert und in einer Wirtszelle exprimiert werden.

11. Prozess zum Herstellen eines Moleküls oder Molekülkomplexes nach einem der Ansprüche 1 bis 7 mithilfe von chemischer Vernetzung.

## Revendications

1. Préparation pharmaceutique comprenant une molécule ou un complexe de molécules capable de se lier à TLR9 et CD32, comprenant au moins un épitope d'au moins un antigène, dans laquelle la partie de la molécule ou du complexe de molécules se liant à CD32 est sélectionnée dans le groupe comprenant les anticorps, les fragments d'anticorps, les domaines protéiques et les peptides, et la partie se liant à TLR9 est un acide nucléique..

2. Préparation pharmaceutique selon la revendication 1, **caractérisée en ce que** l'épitope est un épitope de cellule T.

3. Préparation pharmaceutique selon la revendication 1 ou 2, **caractérisée en ce que** l'épitope provient ou est produit à partir d'au moins un épitope de cellule T contenant un segment d'ADN d'un antigène ou d'un peptide synthétique.

4. Préparation pharmaceutique selon l'une quelconque des revendications 1 à 3, **caractérisée en ce que** l'épitope est sélectionné dans le groupe comprenant les allergènes, spécifiquement les allergènes présents dans la dermatite atopique, l'asthme allergique, la rhinite allergique ou la conjonctivite allergique.

5. Préparation pharmaceutique selon l'une quelconque des revendications 1 à 4, **caractérisée en ce que** l'épitope est isolé à partir d'antigènes complets, d'antigènes dénaturés ou d'antigènes modifiés afin d'empêcher la liaison aux IgE.

6. Préparation pharmaceutique selon l'une quelconque des revendications 1 à 5, **caractérisée en ce qu'**elle comprend au moins un anticorps, un dérivé ou un fragment de celui-ci.

7. Préparation pharmaceutique selon l'une quelconque des revendications 1 à 6 **caractérisée en ce que** la partie se liant à CD32 est au moins une partie d'un anticorps anti-CD32, ou dérivé ou fragment de celui-ci.

8. Préparation pharmaceutique selon l'une quelconque des revendications 1 à 7, pour une utilisation en immunothérapie active.

9. Composition pharmaceutique pour une utilisation selon la revendication 8, pour le traitement ou le traitement prophylactique des allergies.

10. Procédé pour la production d'une molécule ou d'un complexe de molécules selon l'une quelconque des revendications 1 à 7, utilisant une technique recombinante, dans lequel les gènes codant pour les structures de liaison pour TLR9, CD32 et l'épitope sont construits dans un vecteur et exprimés dans une cellule hôte.

11. Procédé pour la production d'une molécule ou d'un complexe de molécules selon l'une quelconque des revendications 1 à 7, utilisant un cross-linking chimique.


### SEQUENCE LISTING

<110> F-STAR BIOTECHNOLOGISCHE FORSCHUNGS- UND ENTWICKLUNGSGES.M.B.H.
<120> Novel molecules
<130> R 49641
<150> EP 06110672.0
   <151> 2006 03 03
<160> 66
<170> PatentIn version 3.3
<210> 1
   <211> 14
   <212> PRT
   <213> human
<400> 1
   Cys Pro Arg His Phe Pro Gln Leu His Pro Asp Thr Phe Ser
   1               5                   10
<210> 2
   <211> 16
   <212> PRT
   <213> human
<400> 2
   Leu Thr His Leu Ser Leu Lys Tyr Asn Asn Leu Thr Val Val Pro Arg
   1               5                   10                  15
<210> 3
   <211> 25
   <212> PRT
   <213> human
<400> 3
   Ala Asn Leu Thr Ala Leu Arg Val Leu Asp Val Gly Gly Asn Cys Arg
   1               5                   10                  15
   Arg Cys Asp His Ala Pro Asn Pro Cys
               20                  25
<210> 4
   <211> 43
   <212> PRT
   <213> Dermatophagoides pteronyssinus
<400> 4
   Gln Ser Cys Arg Arg Pro Asn Ala Gln Arg Phe Gly Ile Ser Asn Tyr
   1               5                   10                  15
   Cys Gln Ile Tyr Pro Pro Asn Ala Asn Lys Ile Arg Glu Ala Leu Ala
               20                  25                  30
   Gln Pro Gln Arg Tyr Cys Arg His Tyr Trp Thr
           35                  40
<210> 5
   <211> 31
   <212> PRT
   <213> Dermatophagoides pteronyssinus
<400> 5
   Gln Ser Cys Arg Arg Pro Asn Ala Gln Arg Phe Gly Ile Ser Asn Tyr
   1               5                   10                  15
   Cys Gln Ile Tyr Pro Pro Asn Ala Asn Lys Ile Arg Glu Ala Leu
               20                  25                  30
<210> 6
   <211> 13
   <212> PRT
   <213> Dermatophagoides pteronyssinus
<400> 6
   Asn Ala Gln Arg Phe Gly Ile Ser Asn Tyr Cys Gln Ile
   1               5                   10
<210> 7
   <211> 25
   <212> PRT
   <213> Dermatophagoides pteronyssinus
<400> 7
   Arg Thr Val Thr Pro Ile Arg Met Gln Gly Gly Cys Gly Ser Cys Trp
   1               5                   10                  15
   Ala Phe Ser Gly Val Ala Ala Thr Glu
               20                  25
<210> 8
   <211> 25
   <212> PRT
   <213> Dermatophagoides pteronyssinus
<400> 8
   Tyr Asp Gly Arg Thr Ile Ile Gln Arg Asp Asn Gly Tyr Gln Pro Asn
   1 5 10 15
   Tyr His Ala Val Asn Ile Val Gly Tyr
   20 25
<210> 9
   <211> 19
   <212> PRT
   <213> Dermatophagoides pteronyssinus
<400> 9
   Pro Cys Ile Ile His Arg Gly Lys Pro Phe Gln Leu Glu Ala Val Phe
   1 5 10 15
   Glu Ala Asn
<210> 10
   <211> 19
   <212> PRT
   <213> Dermatophagoides pteronyssinus
<400> 10
   Lys Tyr Thr Trp Asn Val Pro Lys Ile Ala Pro Lys Ser Glu Asn Val
   1 5 10 15
   Val Val Thr
<210> 11
   <211> 22
   <212> PRT
   <213> Dermatophagoides pteronyssinus
<400> 11
   Glu Asn Val Val Val Thr Val Lys Val Met Gly Asp Asp Gly Val Leu
   1 5 10 15
   Ala Cys Ala Ile Ala Thr
   20
<210> 12
   <211> 22
   <212> PRT
   <213> Dermatophagoides pteronyssinus
<400> 12
   Gln Ser Cys Arg Arg Pro Asn Ala Gln Arg Phe Gly Ile Ser Asn Tyr
   1 5 10 15
   Cys Gln Ile Tyr Pro Pro
   20
<210> 13
   <211> 15
   <212> PRT
   <213> Dermatophagoides pteronyssinus
<400> 13
   Cys Gln Ile Tyr Pro Pro Asn Ala Asn Lys Ile Arg Glu Ala Leu
   1 5 10 15
<210> 14
   <211> 17
   <212> PRT
   <213> Dermatophagoides pteronyssinus
<400> 14
   Ile Arg Glu Ala Leu Ala Gln Pro Gln Arg Tyr Cys Arg His Tyr Trp
   1 5 10 15
   Thr
<210> 15
   <211> 36
   <212> DNA
   <213> human
<400> 15
   aaacgggcag atgctgcacc aactgtatcc atcttc 36
<210> 16
   <211> 164
   <212> PRT
   <213> human
<400> 16
   Cys Gln Ile Tyr Pro Pro Asn Ala Asn Lys Ile Arg Glu Ala Leu Gln
   1               5                   10                  15
   Ser Cys Arg Arg Pro Asn Ala Gln Arg Phe Gly Ile Ser Asn Tyr Cys
               20                  25                  30
   Gln Ile Tyr Pro Pro Tyr Asp Gly Arg Thr Ile Ile Gln Arg Asp Asn
           35                  40                  45
   Gly Tyr Gln Pro Asn Tyr His Ala Val Asn Ile Val Gly Tyr Glu Asn
       50                  55                  60
   Val Val Val Thr Val Lys Val Met Gly Asp Asp Gly Val Leu Ala Cys
   65                  70                  75                  80
   Ala Ile Ala Thr Lys Tyr Thr Trp Asn Val Pro Lys Ile Ala Pro Lys
                   85                  90                  95
   Ser Glu Asn Val Val Val Thr Ile Arg Glu Ala Leu Ala Gln Pro Gln
               100                 105                 110
   Arg Tyr Cys Arg His Tyr Trp Thr Pro Cys Ile Ile His Arg Gly Lys
           115                 120                 125
   Pro Phe Gln Leu Glu Ala Val Phe Glu Ala Asn Arg Thr Val Thr Pro
       130                 135                 140
   Ile Arg Met Gln Gly Gly Cys Gly Ser Cys Trp Ala Phe Ser Gly Val
   145                 150                 155                 160
   Ala Ala Thr Glu
<210> 17
   <211> 28
   <212> DNA
   <213> artificial sequence
<220>
   <223> oligonucleotide for generation of synthetic gene coding for the allergen epitope
<400> 17
   tccggatgcc aaatttaccc gccaaacg 28
<210> 18
   <211> 40
   <212> DNA
   <213> artificial sequence
<220>
   <223> oligonculeotide for generation of synthetic gene coding for allergen epitope
<400> 18
   agcctctctg atcttgttcg cgtttggcgg gtaaatttgg 40
<210> 19
   <211> 40
   <212> DNA
   <213> artificial sequence
<220>
   <223> oligonucleotide for generation of synthetic gene coding for allergen epitope
<400> 19
   cgaacaagat cagagaggct ttgcaatctt gcaggaggcc 40
<210> 20
   <211> 40
   <212> DNA
   <213> artificial sequence
<220>
   <223> oligonucleotide for generation of synthetic gene coding for allergen epitope
<400> 20
   tatgccgaat ctctgcgcat tgggcctcct gcaagattgc 40
<210> 21
   <211> 40
   <212> DNA
   <213> artificial sequence
<220>
   <223> oligonucleotide for generation of synthetic gene coding for allergen epitope
<400> 21
   gcgcagagat tcggcatatc caactactgc cagatctacc 40
<210> 22
   <211> 40
   <212> DNA
   <213> artificial sequence
<220>
   <223> oligonucleotide for generation of synthetic gene coding for allergen epitope
<400> 22
   gtacgcccat cgtatggggg gtagatctgg cagtagttgg 40
<210> 23
   <211> 40
   <212> DNA
   <213> artificial sequence
<220>
   <223> oligonucleotide for generation of synthetic gene coding for allergen epitope
<400> 23
   cccatacgat gggcgtacaa tcatacagcg tgataacggc 40
<210> 24
   <211> 40
   <212> DNA
   <213> artificial sequence
<220>
   <223> oligonucleotide for generation of synthetic gene coding for allergen epitope
<400> 24
   gcgtggtagt taggctgata gccgttatca cgctgtatga 40
<210> 25
   <211> 40
   <212> DNA
   <213> artificial sequence
<220>
   <223> oligonucleotide for generation of synthetic gene coding for allergen epitope
<400> 25
   tatcagccta actaccacgc cgtgaacatc gtcggctacg 40
<210> 26
   <211> 40
   <212> DNA
   <213> artificial sequence
<220>
   <223> oligonucleotide for generation of synthetic gene coding for allergen epitope
<400> 26
   tcacagtaac cacgacattc tcgtagccga cgatgttcac 40
<210> 27
   <211> 40
   <212> DNA
   <213> artificial sequence
<220>
   <223> oligonucleotide for generation of synthetic gene coding for allergen epitope
<400> 27
   agaatgtcgt ggttactgtg aaggtaatgg gcgatgacgg 40
<210> 28
   <211> 40
   <212> DNA
   <213> artificial sequence
<220>
   <223> oligonucleotide for generation of synthetic gene coding for allergen epitope
<400> 28
   agctatggcg caagctagaa ccccgtcatc gcccattacc 40
<210> 29
   <211> 40
   <212> DNA
   <213> artificial sequence
<220>
   <223> oligonucleotide for generation of synthetic gene coding for allergen epitope
<400> 29
   tctagcttgc gccatagcta ccaagtacac ttggaacgta 40
<210> 30
   <211> 40
   <212> DNA
   <213> artificial sequence
<220>
   <223> oligonucleotide for generation of synthetic gene coding for allergen epitope
<400> 30
   ttttcggcgc aattttgggt acgttccaag tgtacttggt 40
<210> 31
   <211> 40
   <212> DNA
   <213> artificial sequence
<220>
   <223> oligonucleotide for generation of synthetic gene coding for allergen epitope
<400> 31
   cccaaaattg cgccgaaaag tgaaaacgtc gtagtgacca 40
<210> 32
   <211> 40
   <212> DNA
   <213> artificial sequence
<220>
   <223> oligonucleotide for generation of synthetic gene coding for allergen epitope
<400> 32
   tgagccaatg cctcccttat ggtcactacg acgttttcac 40
<210> 33
   <211> 40
   <212> DNA
   <213> artificial sequence
<220>
   <223> oligonucleotide for generation of synthetic gene coding for allergen epitope
<400> 33
   agggaggcat tggctcaacc tcaaagatac tgcagacact 40
<210> 34
   <211> 40
   <212> DNA
   <213> artificial sequence
<220>
   <223> oligonucleotide for generation of synthetic gene coding for allergen epitope
<400> 34
   ttatgcaggg cgtccagtag tgtctgcagt atctttgagg 40
<210> 35
   <211> 40
   <212> DNA
   <213> artificial sequence
<220>
   <223> oligonucleotide for generation of synthetic gene coding for allergen epitope
<400> 35
   actggacgcc ctgcataatc caccgtggta aaccctttca 40
<210> 36
   <211> 40
   <212> DNA
   <213> artificial sequence
<220>
   <223> oligonucleotide for generation of synthetic gene coding for allergen epitope '
<400> 36
   cttcgaacac tgcctcaagt tgaaagggtt taccacggtg 40
<210> 37
   <211> 40
   <212> DNA
   <213> artificial sequence
<220>
   <223> oligonucleotide for generation of synthetic gene coding for allergen epitope
<400> 37
   acttgaggca gtgttcgaag ctaacaggac ggtaacgcca 40
<210> 38
   <211> 40
   <212> DNA
   <213> artificial sequence
<220>
   <223> oligonucleotide for generation of synthetic gene coding for allergen epitope
<400> 38
   ccgcacccac cttgcatacg aattggcgtt accgtcctgt 40
<210> 39
   <211> 40
   <212> DNA
   <213> artificial sequence
<220>
   <223> oligonucleotide for generation of synthetic gene coding for allergen epitope
<400> 39
   tgcaaggtgg gtgcgggtct tgttgggctt tttctggtgt 40
<210> 40
   <211> 42
   <212> DNA
   <213> artificial sequence
<220>
   <223> oligonucleotide for generation of synthetic gene coding for allergen epitope
<400> 40
   actagtttat tcagtagcag ccacaccaga aaaagcccaa ca 42
<210> 41
   <211> 6
   <212> PRT
   <213> human
<400> 41
   Lys Arg Ala Pro Arg Glu
   1               5
<210> 42
   <211> 18
   <212> DNA
   <213> artificial sequence
<220>
   <223> silent mutation to introduce unique xhoI site
<400> 42
   aaacgggctc ctcgagaa 18
<210> 43
   <211> 219
   <212> PRT
   <213> artificial sequence
<220>
   <223> complete sequence of a VL-CH3 fusion protein
<400> 43
   Asp Ile Val Met Thr Gln Ala Ala Pro Ser Val Pro Val Thr Pro Gly
   1               5                   10                  15
   Glu Ser Val Ser Ile Ser Cys Arg Ser Ser Lys Ser Leu Leu His Thr
               20                  25                  30
   Asn Gly Asn Thr Tyr Leu His Trp Phe Leu Gln Arg Pro Gly Gln Ser
           35                  40                  45
   Pro Gln Leu Leu Ile Tyr Arg Met Ser Val Leu Ala Ser Gly Val Pro
       50                  55                  60
   Asp Arg Phe Ser Gly Ser Gly Ser Gly Thr Ala Phe Thr Leu Ser Ile
   65                  70                  75                  80
   Ser Arg Val Glu Ala Glu Asp Val Gly Val Phe Tyr Cys Met Gln His
                   85                  90                  95
   Leu Glu Tyr Pro Leu Thr Phe Gly Ala Gly Thr Lys Leu Glu Leu Lys
               100                 105                 110
   Arg Ala Pro Arg Glu Pro Gln Val Tyr Thr Leu Pro Pro Ser Arg Asp
           115                 120                 125
   Glu Leu Gly Ile Ala Gln Val Ser Leu Thr Cys Leu Val Lys Gly Phe
       130                 135                 140
   Tyr Pro Ser Asp Ile Ala Val Glu Trp Glu Ser Asn Gly Gln Pro Glu
   145                 150                 155                 160
   Asn Asn Tyr Lys Thr Thr Pro Pro Val Leu Asp Ser Asp Gly Ser Phe
                   165                 170                 175
   Phe Leu Tyr Ser Lys Leu Thr Val Leu Gly Arg Arg Trp Thr Leu Gly
               180                 185                 190
   Asn Val Phe Ser Cys Ser Val Met His Glu Ala Leu His Asn His Tyr
           195                 200                 205
   Thr Gln Lys Ser Leu Ser Leu Ser Pro Gly Lys
       210                 215
<210> 44
   <211> 681
   <212> DNA
   <213> artificial sequence
<220>
   <223> nucleic acid sequence of VL-CH3 fusion protein
<400> 44
   gaattcgaca ttgtgatgac ccaggctgca ccctctgtac ctgtcactcc tggagagtca 60
   gtatccatct cctgcaggtc tagtaagagt ctcctgcata ctaatggcaa cacttacttg 120
   cattggttcc tacagaggcc aggccagtct cctcagctcc tgatatatcg gatgtccgtc 180
   cttgcctcag gagtcccaga caggttcagt ggcagtgggt caggaactgc tttcacactg 240
   agcatcagta gagtggaggc tgaggatgtg ggtgtttttt actgtatgca acatctagaa 300
   tatccgctca cgttcggtgc tgggaccaag ctggaactga aacgggctcc tcgagaacca 360
   caggtgtaca ccctgccccc atcccgggac gagctcggca tcgcgcaagt cagcctgacc 420
   tgcctggtca aaggcttcta tcccagcgac atcgccgtgg agtgggagag caacgggcag 480
   ccggagaaca actacaagac cacgcctccc gtgctggact ccgacggctc tttcttcctc 540
   tacagcaagc ttaccgtgtt gggccgcagg tggaccctgg ggaacgtctt ctcatgctcc 600
   gtgatgcatg aggctctgca caaccactac acacagaaga gcctctccct gtctccgggt 660
   aaatgagggc gcgccggtac c 681
<210> 45
   <211> 6
   <212> PRT
   <213> artificial sequence
<220>
   <223> part of heavy chain of CH3 domain
<400> 45
   Ala Lys Thr Arg Glu Pro
   1               5
<210> 46
   <211> 18
   <212> DNA
   <213> artificial sequence
<220>
   <223> VH-CH3 with silent mutation to introduce xhoI site
<400> 46
   gccaaaactc gagaacca 18
<210> 47
   <211> 250
   <212> PRT
   <213> artificial sequence
<220>
   <223> complete sequence of VH-CH3 fusion protein
<400> 47
   Glu Val Gln Leu Gln Gln Ser Gly Pro Glu Leu Lys Lys Pro Gly Glu
   1               5                   10                  15
   Thr Val Lys Ile Ser Cys Lys Ala Ser Gly Tyr Thr Phe Thr Asn Tyr
               20                  25                  30
   Gly Met Asn Trp Val Lys Gln Ala Pro Gly Lys Gly Leu Lys Trp Met
           35                  40                  45
   Gly Trp Leu Asn Thr Tyr Thr Gly Glu Ser Ile Tyr Pro Asp Asp Phe
       50                  55                  60
   Lys Gly Arg Phe Ala Phe Ser Ser Glu Thr Ser Ala Ser Thr Ala Tyr
   65                  70                  75                  80
   Leu Gln Ile Asn Asn Leu Lys Asn Glu Asp Met Ala Thr Tyr Phe Cys
                   85                  90                  95
   Ala Arg Gly Asp Tyr Gly Tyr Asp Asp Pro Leu Asp Tyr Trp Gly Gln
               100                 105                 110
   Gly Thr Ser Val Thr Val Ser Ser Ala Lys Thr Arg Glu Pro Gln Val
           115                 120                 125
   Tyr Thr Leu Pro Pro Ser Arg Asp Glu Leu Gly Ile Ala Gln Val Ser
       130                 135                 140
   Leu Thr Cys Leu Val Lys Gly Phe Tyr Pro Ser Asp Ile Ala Val Glu
   145                 150                 155                 160
   Trp Glu Ser Asn Gly Gln Pro Glu Asn Asn Tyr Lys Thr Thr Pro Pro
                   165                 170                 175
   Val Leu Asp Ser Asp Gly Ser Phe Phe Leu Tyr Ser Lys Leu Thr Val
               180                 185                 190
   Leu Gly Arg Arg Trp Thr Leu Gly Asn Val Phe Ser Cys Ser Val Met
           195                 200                 205
   His Glu Ala Leu His Asn His Tyr Thr Gln Lys Ser Leu Ser Leu Ser
       210                 215                 220
   Pro Gly Lys Ser Leu Glu Gln Lys Leu Ile Ser Glu Glu Asp Leu Asn
   225                 230                 235                 240
   Ser Ala Val Asp His His His His His His
                   245                 250
<210> 48
   <211> 759
   <212> DNA
   <213> artificial sequence
<220>
   <223> nucleic acid sequence of VH-CH3 complete fusion protein
<400> 48
   gaattcgagg ttcagcttca gcagtctgga cctgagctga agaagcctgg agagacagtc     60
   aagatctcct gcaaggcttc tgggtatacc ttcacaaact atggaatgaa ctgggtgaag    120
   caggctccag gaaagggttt aaagtggatg ggctggttaa acacctacac tggagagtca    180
   atatatcctg atgacttcaa gggacggttt gccttctctt cggaaacctc tgccagcact    240
   gcctatttgc agatcaacaa cctcaaaaat gaggacatgg ctacatattt ctgtgcaaga    300
   ggggactatg gttacgacga ccctttggac tactggggtc aaggaacctc agtcaccgtc    360
   tcctcagcca aaactcgaga accacaggtg tacaccctgc ccccatcccg ggacgagctc    420
   ggcatcgcgc aagtcagcct gacctgcctg gtcaaaggct tctatcccag cgacatcgcc    480
   gtggagtggg agagcaacgg gcagccggag aacaactaca agaccacgcc tcccgtgctg    540
   gactccgacg gctctttctt cctctacagc aagcttaccg tgttgggccg caggtggacc    600
   ctggggaacg tcttctcatg ctccgtgatg catgaggctc tgcacaacca ctacacacag    660
   aagagcctct ccctgtctcc gggtaaatct ctagaacaaa aactcatctc agaagaggat    720
   ctgaatagcg ccgtcgacca tcatcatcat catcattga                           759
<210> 49
   <211> 30
   <212> DNA
   <213> artificial sequence
<220>
   <223> primer 4.3HupEco for PCR amplification
<400> 49
   cagagaattc gaggttcagc ttcagcagtc 30
<210> 50
   <211> 30
   <212> DNA
   <213> artificial sequence
<220>
   <223> primer 4.3HDOWNXHO for PCR amplification
<400> 50
   gatgctcgag ttttggctga ggagacggtg 30
<210> 51
   <211> 32
   <212> DNA
   <213> artificial sequence
<220>
   <223> primer CH3UPXHOA for PCR amplification
<400> 51
   aaaactcgag aaccacaggt gtacaccctg cc 32
<210> 52
   <211> 34
   <212> DNA
   <213> artificial sequence
<220>
   <223> primer CH3XBA2 for PCR amplification
<400> 52
   actgatctag acctttaccc ggagacaggg agag 34
<210> 53
   <211> 32
   <212> DNA
   <213> artificial sequence
<220>
   <223> primer 4.3LUPECO for PCR amplification
<400> 53
   gatagaattc gacattgtga tgacccaggc tg 32
<210> 54
   <211> 31
   <212> DNA
   <213> artificial sequence
<220>
   <223> primer 4.3LDOWNXHO for PCR amplification
<400> 54
   attactcgag gagcccgttt cagttccagc t 31
<210> 55
   <211> 32
   <212> DNA
   <213> artificial sequence
<220>
   <223> primer CH3UPXHOB for PCR amplification
<400> 55
   gctcctcgag aaccacaggt gtacaccctg cc 32
<210> 56
   <211> 43
   <212> DNA
   <213> artificial sequence
<220>
   <223> primer CH3STOPKPN for PCR amplification
<400> 56
   acgtggtacc tcaggcgcgc cctttacccg gagacaggga gag 43
<210> 57
   <211> 32
   <212> DNA
   <213> artificial sequence
<220>
   <223> primer EpiTLR1 for PCR amplification
<400> 57
   taaagggcgc gcctccggat gccaaattta cc 32
<210> 58
   <211> 35
   <212> DNA
   <213> artificial sequence
<220>
   <223> primer EpiTLR2 for PCR amplification
<400> 58
   tacctcaggc gcgccttatt cagtagcagc cacac 35
<210> 59
   <211> 114
   <212> PRT
   <213> artificial sequence
<220>
   <223> recombinantly produced antibody
<400> 59
   Asp Ile Val Met Thr Gln Ala Ala Pro Ser Val Pro Val Thr Pro Gly
   1 5 10 15
   Glu Ser Val Ser Ile Ser Cys Arg Ser Ser Lys Ser Leu Leu His Thr
   20 25 30
   Asn Gly Asn Thr Tyr Leu His Trp Phe Leu Gln Arg Pro Gly Gln Ser
   35 40 45
   Pro Gln Leu Leu Ile Tyr Arg Met Ser Val Leu Ala Ser Gly Val Pro
   50 55 60
   Asp Arg Phe Ser Gly Ser Gly Ser Gly Thr Ala Phe Thr Leu Ser Ile
   65 70 75 80
   Ser Arg Val Glu Ala Glu Asp Val Gly Val Phe Tyr Cys Met Gln His
   85 90 95
   Leu Glu Tyr Pro Leu Thr Phe Gly Ala Gly Thr Lys Leu Glu Leu Lys
   100 105 110
   Arg Ala
<210> 60
   <211> 342
   <212> DNA
   <213> artificial sequence
<220>
   <223> nucleic acid sequence of recombinantly produced antibody
<400> 60
   gacattgtga tgacccaggc tgcaccctct gtacctgtca ctcctggaga gtcagtatcc      60
   atctcctgca ggtctagtaa gagtctcctg catactaatg gcaacactta cttgcattgg     120
   ttcctacaga ggccaggcca gtctcctcag ctcctgatat atcggatgtc cgtccttgcc     180
   tcaggagtcc cagacaggtt cagtggcagt gggtcaggaa ctgctttcac actgagcatc     240
   agtagagtgg aggctgagga tgtgggtgtt ttttactgta tgcaacatct agaatatccg     300
   ctcacgttcg gtgctgggac caagctggaa ctgaaacggg ct                        342
<210> 61
   <211> 123
   <212> PRT
   <213> artificial sequence
<220>
   <223> recombinantly produce antibody
<400> 61
   Glu Val Gln Leu Gln Gln Ser Gly Pro Glu Leu Lys Lys Pro Gly Glu
   1               5                   10                  15
   Thr Val Lys Ile Ser Cys Lys Ala Ser Gly Tyr Thr Phe Thr Asn Tyr
               20                  25                  30
   Gly Met Asn Trp Val Lys Gln Ala Pro Gly Lys Gly Leu Lys Trp Met
           35                  40                  45
   Gly Trp Leu Asn Thr Tyr Thr Gly Glu Ser Ile Tyr Pro Asp Asp Phe
       50                  55                  60
   Lys Gly Arg Phe Ala Phe Ser Ser Glu Thr Ser Ala Ser Thr Ala Tyr
   65                  70                  75                  80
   Leu Gln Ile Asn Asn Leu Lys Asn Glu Asp Met Ala Thr Tyr Phe Cys
                   85                  90                  95
   Ala Arg Gly Asp Tyr Gly Tyr Asp Asp Pro Leu Asp Tyr Trp Gly Gln
               100                 105                 110
   Gly Thr Ser Val Thr Val Ser Ser Ala Lys Thr
           115                 120
<210> 62
   <211> 369
   <212> DNA
   <213> artificial sequence
<220>
   <223> nucleic acid sequence of recombinantly produced antibody
<400> 62
   gaggttcagc ttcagcagtc tggacctgag ctgaagaagc ctggagagac agtcaagatc      60
   tcctgcaagg cttctgggta taccttcaca aactatggaa tgaactgggt gaagcaggct     120
   ccaggaaagg gtttaaagtg gatgggctgg ttaaacacct acactggaga gtcaatatat     180
   cctgatgact tcaagggacg gtttgccttc tcttcggaaa cctctgccag cactgcctat     240
   ttgcagatca acaacctcaa aaatgaggac atggctacat atttctgtgc aagaggggac     300
   tatggttacg acgacccttt ggactactgg ggtcaaggaa cctcagtcac cgtctcctca     360
   gccaaaaca                                                             369
<210> 63
   <211> 6537
   <212> DNA
   <213> artificial sequence
<220>
   <223> final expression vector containing TLR9 and CD32 binding regions
<400> 63
   agatctaaca tccaaagacg aaaggttgaa tgaaaccttt ttgccatccg acatccacag      60
   gtccattctc acacataagt gccaaacgca acaggagggg atacactagc agcagaccgt     120
   tgcaaacgca ggacctccac tcctcttctc ctcaacaccc acttttgcca tcgaaaaacc     180
   agcccagtta ttgggcttga ttggagctcg ctcattccaa ttccttctat taggctacta     240
   acaccatgac tttattagcc tgtctatcct ggcccccctg gcgaggttca tgtttgttta      300
   tttccgaatg caacaagctc cgcattacac ccgaacatca ctccagatga gggctttctg     360
   agtgtggggt caaatagttt catgttcccc aaatggccca aaactgacag tttaaacgct     420
   gtcttggaac ctaatatgac aaaagcgtga tctcatccaa gatgaactaa gtttggttcg     480
   ttgaaatgct aacggccagt tggtcaaaaa gaaacttcca aaagtcggca taccgtttgt     540
   cttgtttggt attgattgac gaatgctcaa aaataatctc attaatgctt agcgcagtct 600
   ctctatcgct tctgaacccc ggtgcacctg tgccgaaacg caaatgggga aacacccgct 660
   ttttggatga ttatgcattg tctccacatt gtatgcttcc aagattctgg tgggaatact 720
   gctgatagcc taacgttcat gatcaaaatt taactgttct aacccctact tgacagcaat 780
   atataaacag aaggaagctg ccctgtctta aacctttttt tttatcatca ttattagctt 840
   actttcataa ttgcgactgg ttccaattga caagcttttg attttaacga cttttaacga 900
   caacttgaga agatcaaaaa acaactaatt attcgaaacg atgagatttc cttcaatttt 960
   tactgctgtt ttattcgcag catcctccgc attagctgct ccagtcaaca ctacaacaga 1020
   agatgaaacg gcacaaattc cggctgaagc tgtcatcggt tactcagatt tagaagggga 1080
   tttcgatgtt gctgttttgc cattttccaa cagcacaaat aacgggttat tgtttataaa 1140
   tactactatt gccagcattg ctgctaaaga agaaggggta tctctcgaga aaagagaggc 1200
   tgaagctgaa ttcgaggttc agcttcagca gtctggacct gagctgaaga agcctggaga 1260
   gacagtcaag atctcctgca aggcttctgg gtataccttc acaaactatg gaatgaactg 1320
   ggtgaagcag gctccaggaa agggtttaaa gtggatgggc tggttaaaca cctacactgg 1380
   agagtcaata tatcctgatg acttcaaggg acggtttgcc ttctcttcgg aaacctctgc 1440
   cagcactgcc tatttgcaga tcaacaacct caaaaatgag gacatggcta catatttctg 1500
   tgcaagaggg gactatggtt acgacgaccc tttggactac tggggtcaag gaacctcagt 1560
   caccgtctcc tcagccaaaa ctcgagaacc acaggtgtac accctgcccc catcccggga 1620
   tgagctgggc atcgcgcaag tcagcctgac ctgcctggtc aaaggcttct atcccagcga 1680
   catcgccgtg gagtgggaga gcaacgggca gccggagaac aactacaaga ccacgcctcc 1740
   cgtgctggac tccgacggct ctttcttcct ctacagcaag cttaccgtgt tgggccgcag 1800
   gtggaccctg gggaacgtct tctcatgctc cgtgatgcat gaggctctgc acaaccacta 1860
   cacgcagaag agcctctccc tgtctccggg taaatctcta gaacaaaaac tcatctcaga 1920
   agaggatctg aatagcgccg tcgaccatca tcatcatcat cattgagttt gtagccttag 1980
   acatgactgt tcctcagttc aagttgggca cttacgagaa gaccggtctt gctagattct 2040
   aatcaagagg atgtcagaat gccatttgcc tgagagatgc aggcttcatt tttgatactt 2100
   ttttatttgt aacctatata gtataggatt ttttttgtca ttttgtttct tctcgtacga 2160
   gcttgctcct gatcagccta tctcgcagct gatgaatatc ttgtggtagg ggtttgggaa 2220
   aatcattcga gtttgatgtt tttcttggta tttcccactc ctcttcagag tacagaagat 2280
   taagtgagac cttcgtttgt gcagatccaa catccaaaga cgaaaggttg aatgaaacct 2340
   ttttgccatc cgacatccac aggtccattc tcacacataa gtgccaaacg caacaggagg 2400
   ggatacacta gcagcagacc gttgcaaacg caggacctcc actcctcttc tcctcaacac 2460
   ccacttttgc catcgaaaaa ccagcccagt tattgggctt gattggagct cgctcattcc 2520
   aattccttct attaggctac taacaccatg actttattag cctgtctatc ctggcccccc 2580
   tggcgaggtt catgtttgtt tatttccgaa tgcaacaagc tccgcattac acccgaacat 2640
   cactccagat gagggctttc tgagtgtggg gtcaaatagt ttcatgttcc ccaaatggcc 2700
   caaaactgac agtttaaacg ctgtcttgga acctaatatg acaaaagcgt gatctcatcc 2760
   aagatgaact aagtttggtt cgttgaaatg ctaacggcca gttggtcaaa aagaaacttc 2820
   caaaagtcgg cataccgttt gtcttgtttg gtattgattg acgaatgctc aaaaataatc 2880
   tcattaatgc ttagcgcagt ctctctatcg cttctgaacc ccggtgcacc tgtgccgaaa 2940
   cgcaaatggg gaaacacccg ctttttggat gattatgcat tgtctccaca ttgtatgctt 3000
   ccaagattct ggtgggaata ctgctgatag cctaacgttc atgatcaaaa tttaactgtt 3060
   ctaaccccta cttgacagca atatataaac agaaggaagc tgccctgtct taaacctttt 3120
   tttttatcat cattattagc ttactttcat aattgcgact ggttccaatt gacaagcttt 3180
   tgattttaac gacttttaac gacaacttga gaagatcaaa aaacaactaa ttattcgaaa 3240
   cgatgagatt tccttcaatt tttactgctg ttttattcgc agcatcctcc gcattagctg 3300
   ctccagtcaa cactacaaca gaagatgaaa cggcacaaat tccggctgaa gctgtcatcg 3360
   gttactcaga tttagaaggg gatttcgatg ttgctgtttt gccattttcc aacagcacaa 3420
   ataacgggtt attgtttata aatactacta ttgccagcat tgctgctaaa gaagaagggg 3480
   tatctctcga gaaaagagag gctgaagctg aattcgacat tgtgatgacc caggctgcac 3540
   cctctgtacc tgtcactcct ggagagtcag tatccatctc ctgcaggtct agtaagagtc 3600
   tcctgcatac taatggcaac acttacttgc attggttcct acagaggcca ggccagtctc 3660
   ctcagctcct gatatatcgg atgtccgtcc ttgcctcagg agtcccagac aggttcagtg 3720
   gcagtgggtc aggaactgct ttcacactga gcatcagtag agtggaggct gaggatgtgg 3780
   gtgtttttta ctgtatgcaa catctagaat atccgctcac gttcggtgct gggaccaagc 3840
   tggaactgaa acgggctcct cgagaaccac aggtgtacac cctgccccca tcccgggatg 3900
   agctgggcat cgcgcaagtc agcctgacct gcctggtcaa aggcttctat cccagcgaca 3960
   tcgccgtgga gtgggagagc aacgggcagc cggagaacaa ctacaagacc acgcctcccg 4020
   tgctggactc cgacggctct ttcttcctct acagcaagct taccgtgttg ggccgcaggt 4080
   ggaccctggg gaacgtcttc tcatgctccg tgatgcatga ggctctgcac aaccactaca 4140
   cgcagaagag cctctccctg tctccgggta aagggcgcgc ctgaggtacc tcgagccgcg 4200
   gcggccgcca gctttctaga acaaaaactc atctcagaag aggatctgaa tagcgccgtc 4260
   gaccatcatc atcatcatca ttgagtttgt agccttagac atgactgttc ctcagttcaa 4320
   gttgggcact tacgagaaga ccggtcttgc tagattctaa tcaagaggat gtcagaatgc 4380
   catttgcctg agagatgcag gcttcatttt tgatactttt ttatttgtaa cctatatagt 4440
   ataggatttt ttttgtcatt ttgtttcttc tcgtacgagc ttgctcctga tcagcctatc 4500
   tcgcagctga tgaatatctt gtggtagggg tttgggaaaa tcattcgagt ttgatgtttt 4560
   tcttggtatt tcccactcct cttcagagta cagaagatta agtgagacct tcgtttgtgc 4620
   ggatccccca cacaccatag cttcaaaatg tttctactcc ttttttactc ttccagattt 4680
   tctcggactc cgcgcatcgc cgtaccactt caaaacaccc aagcacagca tactaaattt 4740
   tccctctttc ttcctctagg gtgtcgttaa ttacccgtac taaaggtttg gaaaagaaaa 4800
   aagagaccgc ctcgtttctt tttcttcgtc gaaaaaggca ataaaaattt ttatcacgtt 4860
   tctttttctt gaaatttttt tttttagttt ttttctcttt cagtgacctc cattgatatt 4920
   taagttaata aacggtcttc aatttctcaa gtttcagttt catttttctt gttctattac 4980
   aacttttttt acttcttgtt cattagaaag aaagcatagc aatctaatct aaggggcggt 5040
   gttgacaatt aatcatcggc atagtatatc ggcatagtat aatacgacaa ggtgaggaac 5100
   taaaccatgg ccaagttgac cagtgccgtt ccggtgctca ccgcgcgcga cgtcgccgga 5160
   gcggtcgagt tctggaccga ccggctcggg ttctcccggg acttcgtgga ggacgacttc 5220
   gccggtgtgg tccgggacga cgtgaccctg ttcatcagcg cggtccagga ccaggtggtg 5280
   ccggacaaca ccctggcctg ggtgtgggtg cgcggcctgg acgagctgta cgccgagtgg 5340
   tcggaggtcg tgtccacgaa cttccgggac gcctccgggc cggccatgac cgagatcggc 5400
   gagcagccgt gggggcggga gttcgccctg cgcgacccgg ccggcaactg cgtgcacttc 5460
   gtggccgagg agcaggactg acacgtccga cggcggccca cgggtcccag gcctcggaga 5520
   tccgtccccc ttttcctttg tcgatatcat gtaattagtt atgtcacgct tacattcacg 5580
   ccctcccccc acatccgctc taaccgaaaa ggaaggagtt agacaacctg aagtctaggt 5640
   ccctatttat ttttttatag ttatgttagt attaagaacg ttatttatat ttcaaatttt 5700
   tctttttttt ctgtacagac gcgtgtacgc atgtaacatt atactgaaaa ccttgcttga 5760
   gaaggttttg ggacgctcga aggctttaat ttgcaagctg gagaccaaca tgtgagcaaa 5820
   aggccagcaa aaggccagga accgtaaaaa ggccgcgttg ctggcgtttt tccataggct 5880
   ccgcccccct gacgagcatc acaaaaatcg acgctcaagt cagaggtggc gaaacccgac 5940
   aggactataa agataccagg cgtttccccc tggaagctcc ctcgtgcgct ctcctgttcc 6000
   gaccctgccg cttaccggat acctgtccgc ctttctccct tcgggaagcg tggcgctttc 6060
   tcaatgctca cgctgtaggt atctcagttc ggtgtaggtc gttcgctcca agctgggctg 6120
   tgtgcacgaa ccccccgttc agcccgaccg ctgcgcctta tccggtaact atcgtcttga 6180
   gtccaacccg gtaagacacg acttatcgcc actggcagca gccactggta acaggattag 6240
   cagagcgagg tatgtaggcg gtgctacaga gttcttgaag tggtggccta actacggcta 6300
   cactagaagg acagtatttg gtatctgcgc tctgctgaag ccagttacct tcggaaaaag 6360
   agttggtagc tcttgatccg gcaaacaaac caccgctggt agcggtggtt tttttgtttg 6420
   caagcagcag attacgcgca gaaaaaaagg atctcaagaa gatcctttga tcttttctac 6480
   ggggtctgac gctcagtgga acgaaaactc acgttaaggg attttggtca tgagatc 6537
<210> 64
   <211> 7046
   <212> DNA
   <213> artificial sequence
<220>
   <223> final expression vector containg TLR9 and CD32 binding regions and epitope sequence
<400> 64
<210> 65
   <211> 12
   <212> PRT
   <213> human
<400> 65
   Lys Arg Ala Asp Ala Ala Pro Thr Val Ser Ile Phe
   1               5                   10
<210> 66
   <211> 36
   <212> DNA
   <213> human
<400> 66
   aaacgggctg atgctgcacc aactgtatcc atcttc 36